# EUROPÄISCHE PATENTSCHRIFT

(11) **EP 4 492 594 B1**
(45) Veröffentlichungstag und Bekanntmachung des Hinweises auf die Patenterteilung: **04.02.2026**
(21) Anmeldenummer: 24209162.7
(22) Anmeldetag: 11.04.2023
(51) Int. Cl.: H05H 1/24, A61L 9/18, A61L 9/22, H01T 23/00

(54) **VORRICHTUNG ZUR ATMOSPHÄRISCHEN BARRIEREENTLADUNG, VERWENDUNG DERSELBEN ZUR STECKMONTAGE UND VERFAHREN ZUM BETREIBEN DERSELBEN**
ATMOSPHERIC BARRIER DISCHARGE DEVICE, USE OF SAME FOR PLUG-IN ASSEMBLY AND METHOD FOR OPERATING SAME
DISPOSITIF DE DÉCHARGE À BARRIÈRE ATMOSPHÉRIQUE, SON UTILISATION POUR LE MONTAGE PAR ENFICHAGE ET PROCÉDÉ POUR LE FAIRE FONCTIONNER

(43) Veröffentlichungstag der Anmeldung: 15.01.2025
(62) Teilanmeldung aus: 23720772.5
(73) Patentinhaber: WINTERSTEIGER Dry & Protect GmbH, 6844 Altach (AT)
(72) Erfinder: OBHOLZER, Thomas, 6080 Vill (AT)
(74) Vertreter: Viering, Jentschura & Partner mbB Patent- und Rechtsanwälte

(56) Entgegenhaltungen:
- KR-A- 20180 035 655
- KR-B1- 101 003 729
- KR-B1- 101 233 568
- US-A1- 2003 141 182
- US-A1- 2015 105 716
- US-A1- 2015 351 212
- US-A1- 2018 155 197
- US-A1- 2020 029 414
- ANONYMOUS: "Dielectric Constants", 1 January 2001 (2001-01-01), pages 1 - 1, XP093095210, Retrieved from the Internet <URL:http://hyperphysics.phy-astr.gsu.edu/hbase/Tables/diel.html>
- NAVE CARL ROD: "Dielectric Constants", 1 January 2001 (2001-01-01), pages 1 - 1, XP093095212, Retrieved from the Internet <URL:view-source:http://hyperphysics.phy-astr.gsu.edu/hbase/Tables/diel.html>

## Beschreibung

### Gebiet der Erfindung

Die Erfindung betrifft eine Vorrichtung für eine atmosphärische Barriereentladung und insbesondere eine Elektrodenanordnung zur Erzeugung eines nichtthermischen Plasmas auf Basis einer dielektrischen Barriereentladung (DBD, von engl. "dielectric barrier discharge"), in Form eines Plasmaaktuators, insbesondere zur Behandlung von Oberflächen in einem geschlossenen System (z.B. einer Desinfektionskammer). Ferner betrifft die Erfindung eine Verwendung zur Steckmontage einer einer Vorrichtung zur atmosphärischen Barriereentladung und ein Verfahren zum Betreiben einer Vorrichtung zur atmosphärischen Barriereentladung.

### Hintergrund der Erfindung

Eine dielektrische Barriereentladung (DBD, von engl. "dielectric barrier discharge") ist eine Art von nichtthermischem Plasma, das durch Anlegen einer Wechselspannung an Elektroden erzeugt wird, die durch eine isolierende Schicht (z.B. ein dielektrisches Material) getrennt sind.

Es ist bekannt, dass eine DBD zur Entfernung von Schadstoffen aus Gasen und wässrigen Medien, zur Behandlung von Wunden, zur Behandlung von Oberflächen oder bei Plasmaaktuatoren verwendet werden kann.

Die Erfindung betrifft eine Vorrichtung für eine dielektrisch behinderte Barriereentladung zum simultanen und getrennten Erzeugen von reaktiven Stickstoffspezien (RNS, von engl. "reactive nitrogen species") und reaktiven Sauerstoffspezien (ROS, von engl. "reactive oxygen species") und insbesondere einen Plasmaaktuator, der ohne Gebläse kontinuierlich RNS und ROS beispielsweise in einer Desinfektionskammer freisetzen kann.

Herkömmliche Plasma- bzw. Ionisationsgeräte weisen typischerweise ein Dielektrikum aus Glas, Keramik oder Polymeren auf, auf dessen innerer Oberfläche eine Innenelektrode anliegt. Diese kann beispielsweise aus elektrisch leitfähigen Folien/Schichten, Metallkernen, Geweben oder Edelstahlwolle gebildet sein. An der äußeren Oberfläche des Dielektrikums befindet sich beispielsweise eine Außenelektrode aus einem Gewebe, Gestrick oder Streckgitter, welche geerdet (bspw. mit einem Masse-Potential verbunden) ist.

Durch eine angelegte Wechselspannung an die Innenelektrode entsteht eine dielektrische Barriereentladung (DBD, von engl. "dielectric barrier discharge"), welche reaktive Spezies erzeugt.

Es wurde festgestellt, dass zu den häufig identifizierten Spezies Ozon (O₃), Lachgas (N₂O), Salpetersäure (HNO₃), salpetrige Säure (HNO₂) und Stickstoffdioxid gehören, wobei Ozon mit Entladungen mit geringerer Leistung assoziiert ist/wird und Stickstoffdioxid mit Entladungen höherer Leistung assoziiert ist/wird.

Unter speziellen Bedingungen entstehen in einer Nachglüh-Region nach der Entladung Ozon, Distickstoffoxid, Distickstoffpentoxid (N₂O₅) und Salpetersäure.

Es wurde zudem festgestellt, dass Plasma, welches in einem Hochfrequenz-Niederspannungszustand betrieben wird, eine gleiche antimikrobielle Aktivität wie Plasma hat, welches in einem Niederfrequenz-Hochspannungszustand betrieben wird, wenn die Entladungsleistungsdichte gleich ist.

Die Gasförmig-Konzentrationen von Ozon (O₃) und Stickoxiden (NO und NO₂ oder NO_{X}) werden in den allgemein etablierten "Regimen" für die Chemie einer Barriereentladung quantifiziert: ein Ozon-dominierter Modus mit geringer Leistung; ein von Stickoxiden dominierter Hochleistungsmodus; und eine dazwischenliegende, instabile Übergangsregion.

Es wurde festgestellt, dass die bakterielle Dekontamination an Oberflächen, insbesondere an Geweben, im "NO_{X}-Modus" (d.h. im Stickoxid-Modus) am effektivsten ist, im "Ozon-Modus" weniger effektiv ist und im Übergangsbereich mit den schwächsten antibakteriellen Effekten ist.

Eine repräsentative Publikation aus dem Jahr 2018 ("Antimicrobial Applications of Ambient-Air Plasmas", Universität of California, Berkeley von Matthew John Pavlovich, Dissertation) kommt zu folgenden Feststellungen [1]:
Sowohl Stickoxid als auch Stickstoffdioxid nehmen mit zunehmender Leistung an der Entladungsfläche zu, aber nur oberhalb einer bestimmten Leistungsdichteschwelle, etwa 0,10 W/cm² für NO und 0,20 W/cm² für NO₂. Im Gegensatz dazu wird Ozon nur unterhalb einer Leistungsdichteschwelle von etwa 0,50 W/cm² an der Entladungsfläche beobachtet.

Als Entladungsfläche wird diejenige Fläche an der Außenelektrode verstanden, in der eine geschlossene Entladung auftritt. Diese Fläche kann durch ein typisches Plasmaleuchten unter atmosphärischen Bedingungen festgestellt werden.

Aus DE 10 2019 006 536 B3 ist eine Vorrichtung zur Haut- und insbesondere Wundbehandlung unter Verwendung eines Atmosphärendruck-Plasmas bekannt, welche einen Plasmagenerator aufweist, der wenigstens eine Elektrode, wenigstens ein Dielektrikum, eine elektrische Versorgungseinheit sowie eine Steuereinheit aufweist und ausgebildet ist, das Atmosphärendruck-Plasma mittels einer dielektrischen Barriereentladung zu erzeugen, dadurch gekennzeichnet, dass die Steuereinheit ausgebildet ist, den Plasmagenerator in unterschiedlichen Betriebsmodi derart zu betreiben, dass der wenigstens einen Elektrode in einem ersten Betriebsmodus (M1) eine erste Anregungsenergie, die ausreicht, ein Stickstoffmonoxid (NO)-haltiges Plasma zu erzeugen, und in einem zweiten Betriebsmodus (M2) eine zweite Anregungsenergie, die ausreicht, ein Ozon (O₃)-haltiges Plasma zu erzeugen, zugeführt wird, wobei die erste Anregungsenergie größer ist als die zweite Anregungsenergie, wobei das im ersten Betriebsmodus erzeugte Stickstoffmonoxid-haltige Plasma im Wesentlichen frei von Ozon ist und allenfalls noch Spuren dieser Verbindung enthält, die analytisch praktisch nicht mehr nachweisbar sind, und wobei das im zweiten Betriebsmodus erzeugte Ozon-haltige Plasma im Wesentlichen frei von Stickstoffmonoxid ist und letzteres allenfalls in praktisch nicht mehr nachweisbaren Spuren enthält. In dieser Veröffentlichung erfolgt die Entladung als Oberflächen-Mikroentladung (SMD, von engl. "surface microdischarge").

Aus WO 2022/063446 A1 ist eine Plasmaquelle zur Handdesinfektion bekannt. In einer bevorzugten Ausführungsform betrifft die Erfindung eine Vorrichtung zum Desinfizieren von Körperbereichen aufweisend einen Lüfter zur Erzeugung eines Luftstroms und einen Plasmagenerator, wobei sich der Plasmagenerator im Luftstrom befindet, dadurch gekennzeichnet, dass der Plasmagenerator mindestens einen Plasmastab aufweist, weicher ein dielektrisches Rohr mit einem elektrisch leitenden Kern innerhalb des dielektrischen Rohres aufweist, wobei das dielektrische Rohr auf einer Außenseite einen über zu Windungen gewickelten Draht aufweist, wobei der elektrisch leitende Kern mit dem auf der Außenseite gewickelten Draht ein Elektrodenpaar bildet, welches bei Anlegen einer Spannung ein Plasma erzeugt.

Das dielektrische Rohr weist auf seiner Außenseite einen Draht auf. Dieser Draht wird entlang dieser Außenseite zu Windungen gewickelt. Der elektrisch leitende Kern innerhalb und der zu Windungen gewickelte Draht außerhalb des dielektrischen Rohres bilden ein Elektrodenpaar. Das dielektrische Rohr mit dem Elektrodenpaar wird im Sinne der WO 2022/063446 A1 auch als Plasmastab bezeichnet. Durch das Anlegen einer Spannung, bevorzugt einer Wechselspannung, entsteht die dielektrische Barriereentladung und somit das Plasma und damit die reaktiven Spezies entlang der Oberfläche des dielektrischen Rohres. Vorzugsweise ist der Draht auf der Außenseite des dielektrischen Rohres doppelt und gegenläufig gewickelt. Damit wird die Induktivität, die diese Wicklung haben könnte, kompensiert. Durch die doppelte Wicklung wird ein Aufbau ähnlich wie bei einer Koaxialleitung erreicht, die keine elektromagnetische Strahlung aussendet. Stattdessen können die stromdurchflossenen Wicklungen effektiv zur Erzeugung des Plasmas dienen. Um das dielektrische Rohr gelangt der Luftstrom durch den Lüfter bedingt in Querrichtung.

Aus US 5,581,152 A "Dielectric barrier discharge lamp", ist eine dielektrische Barriere-Entladungslampe, die beispielsweise als Ultraviolett-Lichtquelle für eine photochemische Reaktion verwendet wird und bei der Licht von "Excimer"-Molekülen abgestrahlt wird, die durch eine dielektrische BarriereEntladung gebildet werden, bekannt.

Generell funktionieren Plasmaaktuatoren durch die Erzeugung eines Plasmafelds, das eine ionisierte Gaswolke erzeugt, die auf die Strömung von Gasen oder Flüssigkeiten einwirkt. Das Plasmafeld wird durch Anlegen einer Hochspannung zwischen zwei Elektroden erzeugt, die in einem Dielektrikum angeordnet sind.

Wenn eine Hochspannung anliegt, werden Elektronen von der Elektrode abgestoßen und durch das Gas beschleunigt. Diese Elektronen stoßen dann mit den Atomen und Molekülen im Gas zusammen und ionisieren sie. Dies führt zur Bildung von positiv geladenen Ionen und negativ geladenen Elektronen.

Die ionisierte Gaswolke, die als Plasma bezeichnet wird, erzeugt dann elektrische Kräfte und elektromagnetische Felder, die eine Strömung bewirken. Durch die Steuerung der Hochspannung kann die Form und Intensität des Plasmas und damit die Wirkung auf die Strömung kontrolliert werden.

Plasmaaktuatoren können auch zur Erzeugung von Ozon eingesetzt werden. Wenn ein Plasmaaktuator auf Luft oder Sauerstoff angewendet wird, erzeugt das Plasma eine ionisierte Gaswolke, die reaktive Sauerstoffverbindungen, einschließlich Ozon (O₃), erzeugt.

In JP2009242172A wird ein Ozon-erzeugendes Gerät auf Basis eines Plasmaaktuators beschrieben. Unter Verwendung eines Elektrodenpaares auf der Rückseite und auf den oberflächenseitigen Elektroden und einer Wechselspannung an den beiden Elektroden wird ein Oberflächenplasma am Rand der Oberflächenelektrode erzeugt.

Eine repräsentative Untersuchung aus dem Jahr 2022 (Wang Xi et a. "Mode transition of air surface micro-discharge and its effect on the water activation and antibacterial activity ", 2022 Plasma Sources Sci. Technol.) kam zu dem Ergebnis, dass die Konzentrationen von H⁺, H₂O₂, NO₂⁻, NO₃⁻ und ONOO⁻ für den Stickoxid-Modus etwa 2-3-mal so hoch sind wie die für den Ozon-Modus, während die O₃ -Konzentration für den Ozon-Modus mehr als das 28-fache der im Stickoxid-Modus ist. Es wird auch festgestellt, dass die Auswirkung der Umgebungstemperatur auf den Modus-Übergang konsistent ist zur Entladeleistung. [3]

In einer Untersuchung aus dem Jahr 2022 (Zifeng Wang et al. "Combination of NOX mode and O3 mode air discharges for water activation to produce a potent disinfectant", 2022 Plasma Sources Sci. Technol.) wird festgestellt, dass N₂O₅ effektiv und stabil erzeugt wird, indem Plasma in einem NO_{X}-Modus und in einem O₃-Modus getrennt hergestellt wurde und anschließend vermischt wurde. Dabei wurde Luftplasma im NO_{X}-Modus durch eine Gleitlichtbogenentladung erzeugt und ein Luftplasma im O₃-Modus durch eine dielektrische Barriereentladung erzeugt. Nach einer Mischung der beiden Gase konnten hohe Konzentration an N₂O₅ erreicht werden. Es wird mit dieser Kombination eine Keimreduktion von fast 6 Log-Stufen erreicht. [2]

Aus KR 10-1003729 ist eine Vorrichtung zur Erzeugung von Plasma durch Ionisierung von Sauerstoff in der Luft bekannt.

Aus US 2015/0351212 A1 ist eine Spulenanordnung zur Erzeugung von Plasma auch bekannt.

Die bisherigen Untersuchungen zeigen jedoch, dass es wünschenswert wäre, eine Vorrichtung, eine Verwendung derselben und ein Verfahren zum Betreiben derselben zur Keimreduktion an Oberflächen zur Verfügung zu haben, welche simultan ein Plasma im Stickoxid- und im Ozon-Modus erzeugen kann (z.B. erzeugt), kostengünstig herzustellen ist und gesteuert/geregelt werden kann. Aufgabe der Erfindung ist es dementsprechend, eine solche Vorrichtung, eine solche Verwendung und ein solches Verfahren bereitzustellen.

### Kurzbeschreibung der Figuren

Im Folgenden wird die Erfindung ausführlich unter Bezugnahme auf die Zeichnungen beschrieben.
Fig. 1 stellt die Befestigung einer Außenelektrode an einem hohlzylindrischen Dielektrikum und einer Masseleitung als Draufsicht gemäß einer Ausführungsform schematisch dar.
Fig. 2 stellt eine Vorrichtung zur atmosphärischen Barriereentladung gemäß einer Ausführungsform schematisch dar (rechts: Draufsicht; links: Seitenansicht).
Fig. 3 stellt ein Rechteckmaschen-Gewebe an (z.B. auf) einem Dielektrikum (Außenelektrode) gemäß einer Ausführungsform als Seitenansicht schematisch dar.
Fig. 4 stellt die Geometrie einer Außenelektrode mit Steigungen gemäß einer Ausführungsform schematisch dar.
Fig. 5 stellt eine modulare Anordnung der Außenelektrode am Dielektrikum als Seitenansicht gemäß einer Ausführungsform schematisch dar (oben: spiralförmige Wicklung; unten: modulweise in Segmenten gewickelt).
Fig. 6 stellt eine Ausführungsform mit einer Innenelektrode aus einem Elastomer als perspektivische Ansicht schematisch dar.
Fig. 7 stellt eine Ausführungsform mit einer Innenelektrode aus Maschengewebe als perspektivische Ansicht schematisch dar.
Fig. 8 stellt die Verpressung der Innenelektrode aus Fig. 7 gemäß einer Ausführungsform als Draufsicht schematisch dar.
Fig. 9 stellt einen Reaktionsschrank gemäß einer Ausführungsform schematisch dar.

### Erläuterung der Erfindung

Gemäß einem ersten Aspekt der Erfindung ist eine Vorrichtung zur atmosphärischen Barriereentladung (z.B. in Form eines Plasmaaktuators) bereitgestellt, wobei die Vorrichtung aufweist: mindestens ein hohlzylindrisches Dielektrikum, welches eine innere Mantelfläche sowie eine äußere Mantelfläche hat und welches eine Dielektrizitätskonstante größer 4 hat, eine Steckvorrichtung (z.B. eine Montageplatte), welche mindestens ein (z.B. integral ausgebildetes, z.B. einstückig ausgebildetes, z.B. monolithisch ausgebildetes) Haltemittel aufweist, an (z.B. auf) welchem (Haltemittel) mindestens eine Innenelektrode und mindestens eine Außenelektrode (z.B. durch Einsetzen, z.B. durch Aufschieben) angeordnet (z.B. lösbar und insbesondere zerstörungsfrei montiert) sind und an (z.B. auf) welchem (Haltemittel) das hohlzylindrische Dielektrikum (z.B. durch Einsetzen, z.B. durch Aufschieben) angeordnet (z.B. lösbar und insbesondere zerstörungsfrei montiert) ist, um an der Steckvorrichtung gehalten zu sein/werden, die Innenelektrode, welche innerhalb des hohlzylindrischen Dielektrikums angeordnet ist und (z.B. mit einer Außenfläche davon) an der inneren Mantelfläche anliegt, und eine Außenelektrode, welche ein Rechteckmaschen-Gewebe (d.h. ein Maschengewebe dessen Maschen rechteckig, aber nicht quadratisch sind) aufweist und welche (z.B. mit einer Innenfläche davon) an der äußeren Mantelfläche des Dielektrikums anliegt (z.B. eng anliegt/in Berührung damit ist). Die Steckvorrichtung weist eine plattenförmige Basis auf, an (z.B. auf) welcher das mindestens eine Haltemittel (z.B. integral, z.B. einstückig, z.B. monolithisch) ausgebildet ist. Das mindestens eine Haltemittel weist ein erstes Halteelement auf, welches von der ersten Fläche der plattenförmigen Basis her vorsteht (z.B. und daran integral ausgebildet ist, z.B. daran einstückig ausgebildet ist, z.B. daran monolithisch ausgebildet ist), und das erste Haltemittel dazu eingerichtet ist, die Innenelektrode mit einem Anpressdruck zwischen 10 N/cm² und 50 N/cm² gegen das hohlzylindrische Dielektrikum zu drücken (z.B. zu pressen). Das erste Halteelement ist ein Spannstift. Die Innenelektrode ist aus einem Maschengewebe aus einem paramagnetischen Material gebildet. Zum Beispiel kann das paramagnetische Material eine Aluminiumlegierung (AlMg5) aufweisen. Zum Beispiel kann das Maschengewebe eine Maschenweite w von 0,050 mm bis 0,150 mm und einen Drahtdurchmesser d von 0,05 mm bis 0,14 mm, bevorzugt eine Maschenweite w von 0,075 mm und einen Drahtdurchmesser d von 0,052 mm, haben. Der Spannstift weist einen Längsschlitz (z.B. in einer Richtung orthogonal zur plattenförmigen Basis) auf, die Innenelektrode ist um den Spannstift (z.B. in einer Umfangsrichtung davon) herum gewickelt, um an einer äußeren Mantelfläche des Spannstifts anzuliegen, zwei entgegengesetzte Endabschnitte der Innenelektrode sind in den Längsschlitz eingeführt (z.B. eingesetzt), und das hohlzylindrische Dielektrikum ist um den Spannstift herum so angeordnet, dass es die Innenelektrode gegen die äußere Mantelfläche des Spannstifts drückt.

Die Steckvorrichtung kann ferner einen Permanentmagneten (z.B. einen Permanent-Ringmagneten) aufweisen, welcher koaxial zur Innenelektrode (z.B. in dem hohlzylindrischen Dielektrikum, z.B. in dem hohlzylindrischen Dielektrikum an einem der Steckvorrichtung abgewandten Ende) angeordnet ist (z.B. sodass magnetische Feldlinien des Permanentmagneten Entladungszonen zwischen Außenelektrode und Dielektrikum durchlaufen).

Die plattenförmige Basis kann ferner Montageelemente (z.B. Laschen, z.B. Montagelöcher) aufweisen (z.B. mit welchen die Steckvorrichtung an Rohrleitungen montiert werden kann), welche optional an einem Rand der plattenförmigen Basis angeordnet sind.

Die plattenförmige Basis kann ein Photodiode-Halteelement aufweisen, welches von einer ersten Fläche der plattenförmigen Basis her vorsteht, und die Steckvorrichtung kann ferner eine Photodiode zur Überwachung der atmosphärischen Barriereentladung aufweisen, welche an (z.B. in) dem Photodiode-Halteelement angeordnet ist und welche nach außen hin (d.h. (im Betrieb der Vorrichtung) zum Plasma hin) exponiert ist.

Die plattenförmige Basis kann ein UV-C-emittierende-Vorrichtung-Halteelement aufweisen, welches von einer ersten Fläche der plattenförmigen Basis her vorsteht, und die Steckvorrichtung kann ferner eine UV-C-emittierende Vorrichtung (z.B. eine UV-C-Leuchtdiode) aufweisen, welche an (z.B. in) dem UV-C-emittierende-Vorrichtung-Halteelement angeordnet ist und welche nach außen hin (d.h. (im Betrieb der Vorrichtung) zum Plasma hin) exponiert ist.

Die Innenelektrode kann aus einem temperaturbeständigen leitfähigen Elastomer gebildet sein (z.B. welches einen spezifischen Durchgangswiderstand von 0,2 Ohm*cm bis 0,004 Ohm*cm, bevorzugt 0,008 Ohm*cm, hat).

Die Innenelektrode kann mittels des ersten Halteelements (z.B. flächig und mit gleichmäßigem Druck, z.B. vollflächig) gegen die innere Mantelfläche gedrückt (z.B. gepresst) sein/werden.

Das erste Halteelement kann ein Durchgangsloch (z.B. welches das erste Halteelement und die plattenförmige Basis durchdring) haben, die Vorrichtung kann eine Wechselspannung-Anschlussleitung aufweisen, welche mit der Innenelektrode elektrisch verbunden ist, und die Wechselspannung-Anschlussleitung kann durch das Durchgangsloch des ersten Halteelements hindurch (z.B. nach außen, z.B. nach außen in Bezug auf die Vorrichtung) geführt sein.

Die Vorrichtung kann eine Masse-Anschlussleitung aufweisen, welche mit der Außenelektrode (z.B. an einem Anschlussabschnitt) elektrisch verbunden ist, das mindestens eine Haltemittel kann ein zweites Halteelement aufweisen, welches von der ersten Fläche der plattenförmigen Basis her vorsteht (z.B. und welches mit einem U-förmigen Querschnitt ausgebildet ist; z.B. und daran integral ausgebildet ist, z.B. daran einstückig ausgebildet ist, z.B. daran monolithisch ausgebildet ist), und das zweite Haltemittel kann dazu eingerichtet sein, die Masse-Anschlussleitung mechanisch zu führen (z.B. um eine Position der Außenelektrode zumindest radial nach außen hin und/oder in Umfangsrichtung zu fixieren).

Die Außenelektrode kann als Hohlzylinder (z.B. welcher eine Außenelektrode-Hohlzylinder-Längsachse hat) gebildet sein und kann Querdrähte (welche sich zum Beispiel in Umfangsrichtung um die Außenelektrode-Zylinder-Längsachse herum erstrecken) sowie Längsdrähte (welche sich zum Beispiel parallel zur Außenelektrode-Zylinder-Längsachse erstrecken) aufweisen, die Querdrähte (auch als Kette bezeichnet) können aus einem leitfähigen Material (z.B. mit einem Drahtdurchmesser von 0,25mm bis 0,35 mm, vorzugsweise 0,315 mm) gebildet sein, und die Längsdrähte (auch als Schuss bezeichnet) können aus einem leitfähigen Material (z.B. aus Edelstahl) mit einem Drahtdurchmesser, der 10 % bis 15 % dünner (bevorzugt 12,5 % dünner) als ein/der Drahtdurchmesser der Querdrähte ist, gebildet sein.

Die Querdrähte und die Längsdrähte können das Rechteckmaschen-Gewebe bilden, und ein Abstand benachbarter (z.B. unmittelbar benachbarter, z.B. unmittelbar, ohne dazwischenliegende weitere Querdrähte, benachbarter) Querdrähte kann das 4- bis 6-fache (bevorzugt das 5-fache) eines/des Drahtdurchmessers der Längsdrähte betragen.

Hierdurch haben die Längsdrähte und die Querdrähte im Rechteckmaschen-Gewebe verschiedene Steigungen zum Dielektrikum. Je größer Abstände (Maschenweite) der Querdrähte zueinander sind, desto geringer sind die Winkel der Längsdrähte zum Dielektrikum. Somit ist/wird an den Längsdrähten ein erster Plasmaaktuator erzeugt, was durch eine streifenförmige Entladung festgestellt werden kann. Generell ist bekannt, dass beim einem Plasmaaktuator Elektrodenkanten oder Drähte keinen oder nur einen sehr geringen Winkel gegenüber dem Dielektrikum ausbilden. Bevorzugt beträgt deshalb der Abstand der Querdrähte zueinander das 4- bis 6-fache, bevorzugt das 5-fache des Drahtdurchmessers des Längsdrahtes. Somit ist eine Funktion als Plasmaaktuator gegeben.

Ein Abstand von einem äußersten (z.B. einem letzten) Querdraht, welcher in einem Randabschnitt des Rechteckmaschen-Gewebes in Längsrichtung des Rechteckmaschen-Gewebes angeordnet ist, zu einem jeweiligen freien Ende der Längsdrähte (z.B. das sich über den äußersten (z.B. den letzten) Querdraht in dem Randabschnitt hinauserstreckt) in dem Randabschnitt kann das 0,8- bis 1,5-fache (besonders vorteilhaft das 1,0-fache) des Abstands benachbarter (z.B. unmittelbar benachbarter) Querdrähte des Rechteckmaschen-Gewebes betragen. Hierdurch wird in dem Randabschnitt an dem jeweiligen freien Ende der Längsdrähte ein zweiter Plasmaaktuator erzeugt.

Diese Geometrie gewährleistet in vorteilhafter Weise eine simultane Entladung im Stickoxid-Modus (d.h. im Stickstoffdominierten Modus) und Ozon-Modus (d.h. im Ozon-dominierten Modus). Dabei bilden Abschnittskanten (d.h. die freien Enden im Randabschnitt, z.B. Elektrodenkanten) der Außenelektrode eine nicht thermische "surface micro discharge"-Entladung (SMD-Ozon-Modus) und unterhalb der Außenelektrode bildet sich eine "volume dielectric barrier discharge"-Entladung (VDBD-Stickoxid-Modus).

In Bezug auf die dielektrische Barriereentladung (DBE) im Stickoxid-Modus wurde festgestellt, dass es bestimmte Vorteile von Rechteckmaschen-Geweben (d.h. Maschengeweben deren Maschen rechteckig, aber nicht quadratisch sind) gegenüber Quadratmaschen-Geweben gibt.

Einer der weiteren Vorteile von Rechteckmaschen-Geweben liegt in der Struktur des Gewebes. Im Vergleich zu Quadratmaschen-Geweben hat ein Rechteckmaschen-Gewebe bevorzugt eine höhere Dichte an Drähten, wodurch ein größerer Anteil der Elektrodenoberfläche aktiviert ist/wird. Dies führt zu einer höheren Plasmaleistung und einem höheren Wirkungsgrad der DBE.

Ein weiterer Vorteil von Rechteckmaschen-Geweben ist die Möglichkeit, die Ausrichtung der Drähte zu ändern, um die Richtung des elektrischen Feldes zu steuern. Dies trägt dazu bei, das Plasma gleichmäßiger zu verteilen und Hotspots zu vermeiden, die zu unerwünschten Effekten, wie zum Beispiel der Bildung von zu hohen Ozonkonzentrationen, führen.

Gemäß einem zweiten Aspekt der Erfindung ist eine Verwendung einer oben beschriebenen Vorrichtung zur Steckmontage (z.B. zur Steckmontage als eine einzige Einheit, z.B. zur Steckmontage als eine einzige Einheit ohne Demontieren der Vorrichtung) der Vorrichtung mittels der Steckvorrichtung in einer vordefinieren Öffnung an einem Reaktor (z.B. einem Entkeimungsschrank) bereitgestellt.

Gemäß einem dritten Aspekt der Erfindung ist ein Verfahren zum Betreiben einer oben beschriebenen Vorrichtung zur atmosphärischen Barriereentladung bereitgestellt, wobei wobei die plattenförmige Basis ein Photodiode-Halteelement aufweist, welches von einer ersten Fläche der plattenförmigen Basis her vorsteht, und die Steckvorrichtung ferner eine Photodiode zur Überwachung der atmosphärischen Barriereentladung aufweist, welche an dem Photodiode-Halteelement angeordnet ist und welche nach außen hin exponiert ist und das Verfahren aufweist:
Anlegen einer Wechselspannung mit einem Spannungswert (z.B. einer Spannungsamplitude) und einer Frequenz zwischen der Innenelektrode und der Außenelektrode zum Erzeugen einer atmosphärischen Barriereentladung (z.B. eines Plasmas) an der Außenelektrode; Messen einer UV-Intensität der erzeugten atmosphärischen Barriereentladung mittels der Photodiode; Steuern des Spannungswerts und der Frequenz basierend auf der gemessenen UV-Intensität, sodass die Vorrichtung zur atmosphärischen Barriereentladung im Bereich eines elektrischen Resonanzzustands (z.B. eines elektrischen Resonanzzustands der Vorrichtung zur atmosphärischen Barriereentladung) betrieben wird.

Das Verfahren kann ferner aufweisen: Steuern des Spannungswerts und der Frequenz, sodass in der Vorrichtung zur atmosphärischen Barriereentladung gleichzeitig und räumlich getrennt voneinander ein Ozon-dominiertes Plasma und ein Stickoxid-dominiertes Plasma erzeugt wird.

Das Verfahren kann ferner aufweisen: Betreiben der Vorrichtung zur atmosphärischen Barriereentladung an einem Reaktor (z.B. einem Entkeimungsschrank); Zuführen von gefilterter (z.B. entstaubter) Luft von außerhalb des Reaktors (z.B. aus der Atmosphäre) durch die Vorrichtung zur atmosphärischen Barriereentladung hindurch; Plasma-Behandeln der zugeführten Luft mittels der Vorrichtung zur atmosphärischen Barriereentladung; Zuführen der plasma-behandelten Luft in den Reaktor.

### Ausführliche Beschreibung

Die folgende Beschreibung verschiedener Ausführungsformen der Erfindung ist nicht dazu gedacht, die Erfindung auf irgendeine dieser Ausführungsformen oder korrespondierende Details zu beschränken. Über die Zeichnungen hinweg werden gleiche Bezugszeichen für die gleichen Komponenten verwendet. Begriffe, wie zum Beispiel "erste/erster/erstes", "zweite/zweiter/zweites", etc., sind dazu gedacht, eine korrespondierende Komponente lediglich zu benennen/bezeichnen, ohne eine bestimmte Reihenfolge oder Anzahl von Komponenten zu definieren, sodass eine als eine erste Komponente bezeichnete Komponente auch eine zweite Komponente sein kann und umgekehrt.

Beispielhafte Ausführungsformen werden im Folgenden unter Bezugnahme auf die Figuren beschrieben.

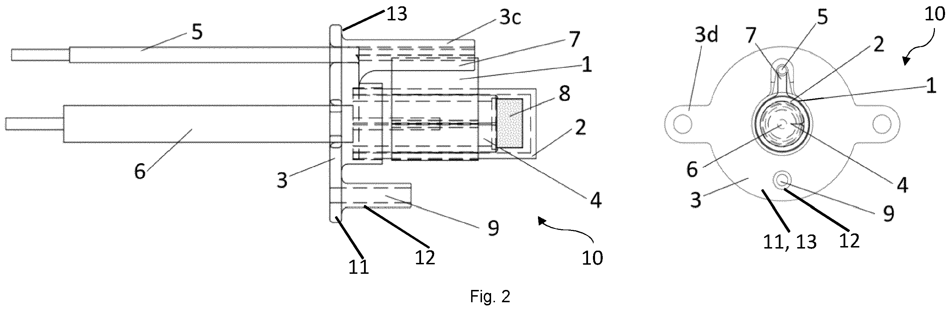

Wie beispielsweise in Fig. 2, Fig. 6, Fig. 7, Fig. 8 und Fig. 1 dargestellt, stellt eine Ausführungsform eine Vorrichtung 10 zur atmosphärischen Barriereentladung bereit, welche aufweist: mindestens ein hohlzylindrisches Dielektrikum 2, welches eine innere Mantelfläche 2a sowie eine äußere Mantelfläche 2b hat und welches eine Dielektrizitätskonstante größer 4 hat, eine Steckvorrichtung 3, welche mindestens ein Haltemittel 3a, 3b, 3c aufweist, an welchem mindestens eine Innenelektrode 4, 4a, 4b und mindestens eine Außenelektrode 1 angeordnet sind und an welchem das hohlzylindrische Dielektrikum 2 angeordnet ist, um an der Steckvorrichtung 3 gehalten zu sein/werden, die Innenelektrode 4, 4a, 4b, welche innerhalb des hohlzylindrischen Dielektrikums 2 angeordnet ist und an der inneren Mantelfläche (des hohlzylindrischen Dielektrikums 2) 2a anliegt, und eine Außenelektrode 1, welche ein Rechteckmaschen-Gewebe 21 aufweist und welche an der äußeren Mantelfläche 2b des hohlzylindrischen Dielektrikums 2 anliegt.

Beispielsweise kann es bevorzugt sein, dass eine Länge der Innenelektrode 4, 4a, 4b (z.B. in einer Längsrichtung/Axialrichtung der Vorrichtung) mindestens doppelt so groß ist wie eine Länge der Außenelektrode 1 (z.B. in der Längsrichtung/Axialrichtung der Vorrichtung).

Die Steckvorrichtung 3 kann so eingerichtet sein, dass sie einfach an bestehenden (z.B. zur Erfindung externen) Rohrleitungen oder diversen (z.B. zur Erfindung externen) Reaktorbauteilen (z.B. Reaktoren) befestigt sein/werden kann.

Die Vorrichtung kann zur Desinfektion und/oder Geruchsneutralisation von Ausrüstungen, Bekleidungen, insbesondere Schuhen (Stiefeln), technischen oder medizinischen Produkten und Gegenständen, Lebensmitteln, Tieren, insbesondere Bienenstöcken, zur Milbenentfernung in einem geschlossenen System verwendet werden.

Die Vorrichtung kann ferner eine elektrische Versorgungseinheit (z.B. zur Spannungszufuhr zur Innenelektrode 4, 4a, 4b und zur Außenelektrode 1) sowie eine Steuereinheit (z.B. zur Steuerung der Vorrichtung 10 und/oder zur Steuerung der Spannungszufuhr zur Vorrichtung 10) aufweisen.

Die Steuereinheit kann so eingerichtet sein, dass sie die Vorrichtung 10 (d.h. den Plasmagenerator) über die Lichtintensität des Plasmas mit einer optimalen Anregungsenergie derart betreiben kann, dass (gleichzeitig) wenigstens ein Bereich der Außenelektrode 1 in einem Stickoxid-Modus (VDPE) und mindestens zwei Bereiche der Außenelektrode 1 in einem Ozon-Modus (SMD, z.B. SMD-Modus) betrieben werden können (z.B. betrieben werden).

Wie beispielsweise in Fig. 2 (linke Seite) dargestellt, kann die Steckvorrichtung 3 ferner einen Permanentmagneten 8 aufweisen, welcher koaxial zur Innenelektrode 4, 4a, 4b angeordnet ist.

In diesem Zusammenhang wurde überraschenderweise festgestellt, dass sich mit einem Permanentmagneten 8 die Ozonkonzentration je nach Anordnung der Pole (Süd- oder Nordpol in Bezug zur Innenelektrode 4, 4a, 4b) erhöhen oder erniedrigen lässt. Es wurde auch festgestellt, dass die Entladungen in einem magnetischen Feld homogener stattfinden. Erklärt wird dies damit, dass die Filamente bei einer DBD nicht gleichmäßig über dem Dielektrikum 2 verteilt sind, sondern aus zahlreichen Mikrofilamenten gebildet sind. Bei Anlegen eines Magnetfeldes ist der Vektor der magnetischen Induktion senkrecht zu den Filamenten. Folglich tritt eine Lorentzkraft auf, welche die Dimension, die Gleichmäßigkeit und die Ausrichtung der Filamente beeinflusst und somit die Ozon- und die Stickoxidkonzentration verändert. Auf dieser Weise trägt die Verwendung eines Magnetfeldes gemäß einer Ausführungsform zu einer Reduzierung von unerwünschten Effekten bei. Vorzugsweise weist die Ionisationsröhre (d.h. die Plasmaröhre) ein Magnetfeld auf, welches vom Nordpol des Permanentmagneten 8 durch die Innenelektrode 4, 4a, 4b und Außenelektrode 1 führt.

Ein weiterer Vorteil ergibt sich dadurch, dass der Plasmakopf (d.h. die Plasmaröhre, z.B. die Vorrichtung 10) ohne weitere Befestigungselemente an einem magnetisierbaren Werkstoff angebracht/befestigt sein/werden kann.

Wie in Fig. 2, Fig. 6, Fig. 7, Fig. 8 und Fig. 1 dargestellt, weist die Steckvorrichtung 3 der erfinderischen Vorrichtung eine plattenförmige Basis 11 auf, an (z.B. auf) welcher das mindestens eine Haltemittel 3a, 3b, 3c ausgebildet ist.

Wie beispielsweise in Fig. 2 (rechte Seite) dargestellt, kann die plattenförmige Basis 11 ferner Montageelemente 3d (z.B. Laschen, z.B. Montagelöcher) aufweisen (z.B. mit welchen die Steckvorrichtung 3 an Rohrleitungen montiert sein/werden kann), welche optional an einem Rand der plattenförmigen Basis 11 angeordnet sind.

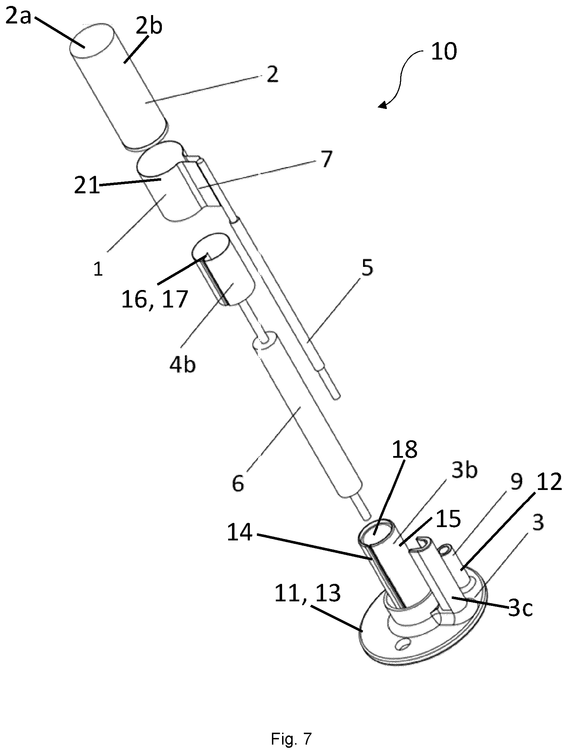

Wie beispielsweise in Fig. 2 (linke Seite) und Fig. 7 dargestellt, kann die plattenförmige Basis 11 ein Photodiode-Halteelement 12 aufweisen, welches von einer ersten Fläche 13 der plattenförmigen Basis 11 her vorsteht, und die Steckvorrichtung 3 kann ferner eine Photodiode 9 zur Überwachung der atmosphärischen Barriereentladung aufweisen, welche an dem Photodiode-Halteelement 12 angeordnet ist und welche nach außen hin exponiert ist.

Die Photodiode 9 kann einfallendes UV-C Licht in elektrischen Strom umwandeln. Aufgrund dieses Photoeffekts kann die Vorrichtung 10 (z.B. der Plasmaaktuator) über die (bspw. mittels der Steuereinheit) zugeführte Spannung gesteuert/geregelt sein/werden. Somit kann die Plasmaintensität, welche neben der Anregungsfrequenz, der Anregungsspannung und der Anregungsstromstärke stark von der Luftfeuchte und der Temperatur abhängt, kontinuierlich gemessen und geregelt werden. Überraschenderweise kann bei atmosphärischen Bedingungen mit einer Photodiode 9 auch die Ozon- und die Negative-IonenKonzentration näherungsweise bestimmt/ermittelt und gesteuert/geregelt werden.

Alternativ dazu kann die plattenförmige Basis 11 ein UV-C-emittierende-Vorrichtung-Halteelement 12 aufweisen, welches von der ersten Fläche 13 der plattenförmigen Basis 11 her vorsteht, und die Steckvorrichtung 3 kann ferner eine UV-C-emittierende Vorrichtung (z.B. eine UV-C-Leuchtdiode, z.B. eine im Wellenlängenbereich von 254 nm bis 280 nm emittierende UV-C-Leuchtdiode) 9 aufweisen, welche an dem UV-C-emittierende-Vorrichtung-Halteelement 12 angeordnet ist und welche nach außen hin (z.B. (im Betrieb der Vorrichtung 10) zum Plasma hin) exponiert ist.

In diesem Zusammenhang zeigen Fig. 2 und Fig. 7 beispielhaft verschiedene Positionen, an denen das Photodiode-Halteelement 12 oder das UV-C-emittierende-Vorrichtung-Halteelement 12 angeordnet sein kann. Die Position davon kann unter anderem in Abhängigkeit davon gewählt sein/werden, welche Luftströmung erzeugt werden soll. Hierbei kann das Photodiode-Halteelement 12 oder das UV-C-emittierende-Vorrichtung-Halteelement 12 vorteilhafterweise zu einer Verwirbelung von Luft beitragen (und damit eine Luftströmung erzeugen und/oder diese beeinflussen).

Generell gilt, dass, wenn eine dielektrische Barriereentladung (DBD) mit einer UV-C-Lampe (z.B. einer UV-C-emittierende Vorrichtung 9) - unter atmosphärischen Bedingungen - mit einem Spektrum von 254 nm bis 280 nm bestrahlt wird, verschiedene chemische Reaktionen stattfinden. Diese Reaktionen sind Teil eines komplexen Prozesses, der als Plasmachemie bezeichnet wird. Unter anderem ist bekannt, dass eine UV-C-Strahlung dazu führt, dass Sauerstoffmoleküle angeregt werden und reaktive Sauerstoffspezies, wie zum Beispiel Singulett-Sauerstoff (O₂*) oder Hydroperoxyl-Radikale (HO₂*), bilden. Hierbei sind gemäß der Ausführungsform sämtliche Anbauteile einer Vorrichtung zur dielektrischen Barriereentladung 10 an (z.B. auf) einer Steckvorrichtung 3 (z.B. integral, z.B. einstückig, z.B. monolithisch) integriert. Hierdurch ergibt sich ein vorteilhafter Effekt, dass die Verwendung der Steckvorrichtung 3 ermöglicht, dünneres Material für die Innenelektrode 4, 4a, 4b im Vergleich zum Stand der Technik zu verwenden, und dass gleichzeitig die Vorrichtung 10 der Ausführungsform schnell und kostengünstig produziert werden kann. Die Verwendung der Steckvorrichtung 3 ermöglicht zudem, dass die Innenelektrode 4, 4a, 4b flächig mit einem gleichmäßigen Druck mittels eines ersten Haltemittels 3a, 3b (z.B. eines Spannstifts) an/gegen das Dielektrikum 2 gedrückt ist/wird.

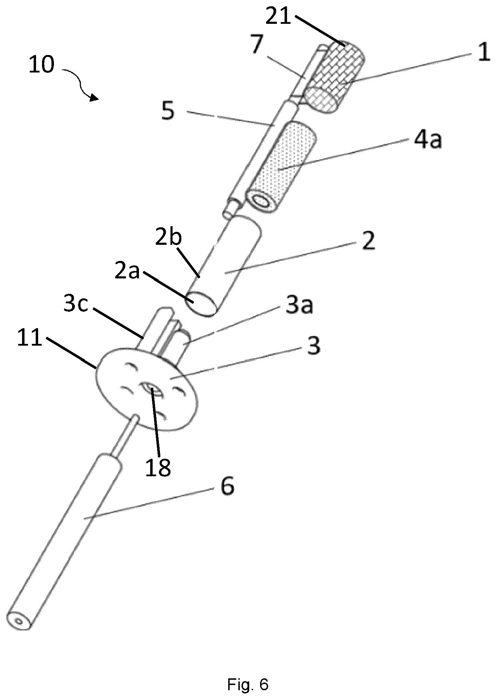

Wie in Fig. 6, Fig. 7 und Fig. 8 dargestellt, weist das mindestens eine Haltemittel 3a, 3b, 3c der erfinderischen Vorrichtung ein erstes Halteelement 3a, 3b aufweisen, welches von der ersten Fläche 13 der plattenförmigen Basis 11 her vorsteht (z.B. und daran integral ausgebildet ist, z.B. daran einstückig ausgebildet ist, z.B. daran monolithisch ausgebildet ist), und das erste Haltemittel 3a, 3b dazu eingerichtet ist, die Innenelektrode 4, 4a, 4b mit einem Anpressdruck zwischen 10 N/cm² und 50 N/cm² gegen das hohlzylindrische Dielektrikum 2 zu drücken. Das erste Halteelement 3a, 3b ist ein Spannstift.

Wie beispielsweise in Fig. 6 dargestellt, kann die Innenelektrode 4, 4a aus einem temperaturbeständigen leitfähigen Elastomer (z.B. aus einem Rund-Hohl-Profil eines temperaturbeständigen leitfähigen Elastomers) gebildet sein. Die Innenelektrode 4, 4a kann beispielsweise ein bis mindestens 125 °C temperaturbeständiges leitfähiges Elastomer auf Silikonbasis, welches einen spezifischen Durchgangswiderstand von 0,2 Ohm*cm bis 0,004 Ohm*cm, besonders bevorzugt 0,008 Ohm*cm aufweist, sein. In einem Montiert-Zustand ist/ wird die Innenelektrode 4, 4a mittels des ersten Halteelements 3a (z.B. vollflächig) gegen die innere Mantelfläche 2a gedrückt.

Beispielsweise ist ein Außendurchmesser des Elastomers (d.h. der Innenelektrode 4, 4a) vor dem Montieren (d.h. Spannen) 5 % bis 10 %, besonders bevorzugt 5 % bis 6 %, geringer als ein Innendurchmesser des zylinderförmigen Dielektrikums 2. Homogen verteilte, leitfähige Feststoffpartikel im Elastomer (d.h. in der Innenelektrode 4, 4a) können Silber beschichtete Kugeln aus Nickel, Kupfer, Aluminium oder Glas, besonders bevorzugt Kupfer, sein. Die Herstellung des leitfähigen Elastomers kann beispielsweise durch Extrusion erfolgen; dabei werden die leitfähigen Feststoffpartikel bahnförmig in axialer Richtung ausgerichtet, was sich positiv auf eine Verlängerung der Filamente im SMD-Bereich auswirkt.

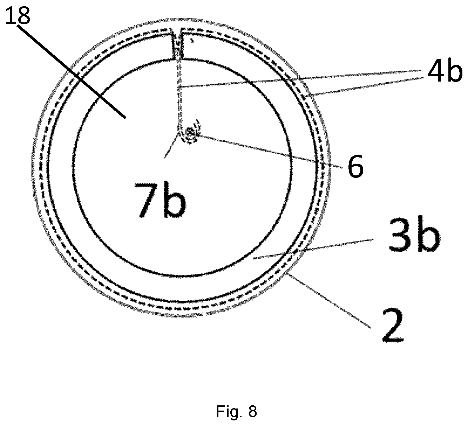

Wie in Fig. 7 und Fig. 8 dargestellt, ist die Innenelektrode 4, 4b der erfinderischen Vorrichtung aus einem Maschengewebe aus einem paramagnetischen Material gebildet. Hierbei kann die Innenelektrode 4, 4b beispielsweise ein feinstrukturiertes Drahtgewebe aus einem paramagnetischen Material aufweisen, welches aus einer Aluminiumlegierung (AlMg5) gebildet sein kann. Bevorzugt ist beispielsweise eine Maschenweite von w 0,098 mm bis 0,150 mm und ein Drahtdurchmesser d von 0,05 mm bis 0,14mm, besonders bevorzugt ist eine Maschenweite von 0,075 mm und ein Drahtdurchmesser von 0,052 mm. Es gilt, dass die Elektronentemperatur des erzeugten Plasmas mit einer Aluminium-Innenelektrode 4, 4b aufgrund der gleichmäßigen elektrischen Entladung (laminare Ionisation) kleiner sein kann als bei anderen bei Verwendung von Ag-Innenelektroden, Cu-Innenelektroden und Edelstahl-Innenelektroden. Es wurde festgestellt, dass bei Verwendung von Al-Innenelektroden 4, 4a die reaktiven Spezies in höheren Konzentrationen - in Bezug auf die Intensitäten, Arten von Ionen und die Anzahl von Ionen - generiert werden als die bei Verwendung von Ag-Innenelektroden, und Cu-Innenelektroden und Edelstahl-Innenelektroden. Mit dieser Elektrodenkonfiguration aus Rechteckmaschen-Gewebe 21 und Aluminium-Maschenelektrode wird bevorzugt ein Verhältnis von negativen Ionen zu positiven Ionen von 0,9 bis 1,1 erreicht.

Gemäß der Erfindung weist der Spannstift 3b einen Längsschlitz 14 auf, ist die Innenelektrode 4, 4b um den Spannstift 3b gewickelt/gewunden, um an einer äußeren Mantelfläche 15 des Spannstifts 3b anzuliegen, sind zwei entgegengesetzte Endabschnitte 16, 17 der Innenelektrode 4, 4b in den Längsschlitz 14 eingeführt, ist/wird das hohlzylindrische Dielektrikum 2 um den Spannstift 3b herum so angeordnet, dass es die Innenelektrode 4, 4b gegen die äußere Mantelfläche 15 des Spannstifts 3b drückt.

Wie in Fig. 6 und Fig. 7 dargestellt, kann das erste Halteelement 3a, 3b ein Durchgangsloch 18 haben. Das Durchgangsloch kann das erste Halteelement 3a, 3b entlang einer Längsrichtung davon durchdringen. Ferner kann das Durchgangsloch 18 die Steckvorrichtung 3 sowie die plattenförmige Basis 11 durchdringen.

Ferner kann die Vorrichtung 10 eine Wechselspannung-Anschlussleitung 6 aufweisen, welche mit der Innenelektrode 4, 4a, 4b elektrisch verbunden ist. Die Wechselspannung-Anschlussleitung 6 kann mit einer externen Spannungsversorgung verbunden sein, um der Innenelektrode 4, 4a, 4b eine Wechselspannung zuzuführen.

Die Wechselspannung-Anschlussleitung 6 kann durch das Durchgangsloch 18 des ersten Halteelements 3a, 3b hindurch geführt sein. Dadurch kann ermöglicht sein/werden, dass die Innenelektrode 4, 4a, 4b von außen (z.B. von der elektrischen Versorgungseinheit, z.B. von der elektrischen Versorgungseinheit und gesteuert mittels der Steuereinheit) mit einer Spannung versorgt ist/wird.

Wie in Fig. 6, Fig. 7, Fig. 2 und Fig. 1 dargestellt, kann die Vorrichtung 10 eine Masse-Anschlussleitung 5 aufweisen, welche mit der Außenelektrode 1 elektrisch verbunden ist. Hierzu kann die Masse-Anschlussleitung 5 mit einer Spannkraft von 150 N/cm² bis 250 N/cm², besonders bevorzugt mit 200 N/cm², mit einem Anschlussabschnitt 7 der Außenelektrode 1 verpresst und daran fixiert sein. Die Masse-Anschlussleitung 5 kann geerdet sein und/oder mit einem Erdpotential verbunden sein.

Das mindestens eine Haltemittel 3a, 3b, 3c kann ein zweites Halteelement 3c aufweisen, welches von der ersten Fläche 13 der plattenförmigen Basis 11 her vorsteht. Das zweite Haltemittel 3c kann dazu eingerichtet sein, die Masse-Anschlussleitung 5 mechanisch zu führen. Das zweite Halteelement 3c kann mit einem U-förmigen Querschnitt ausgebildet sein, welcher beispielsweise in Richtung des ersten Halteelements 3a, 3b offen ist. Hierdurch kann, wenn die Masse-Anschlussleitung 5 in das zweite Halteelement 3c eingesetzt (z.B. eingeführt) ist/wird, das zweite Haltelement 3c eine Position der Außenelektrode 1 zumindest radial nach außen hin und/oder in Umfangsrichtung fixieren. Wie auch mittels des Photodiode-Halteelements 12 oder des UV-C-emittierende-Vorrichtung-Halteelements 12 kann mittels des zweiten Halteelements 3c eine zusätzliche Luftverwirbelung hervorgerufen werden, was die entsprechenden Effekte mittels des Photodiode-Halteelements 12 oder des UV-C-emittierende-Vorrichtung-Halteelements 12 verstärken kann.

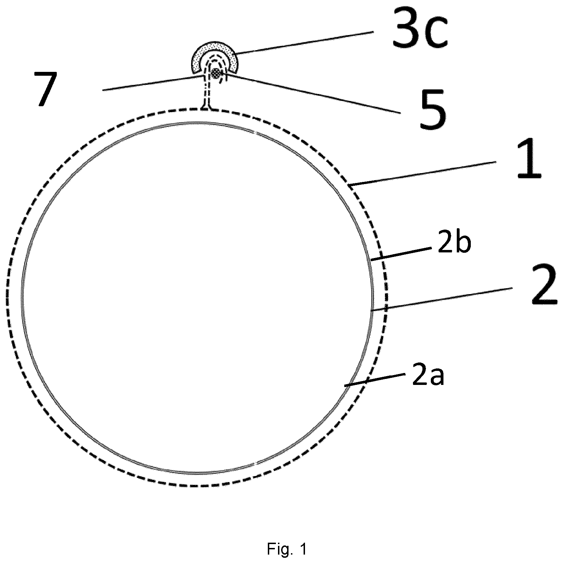

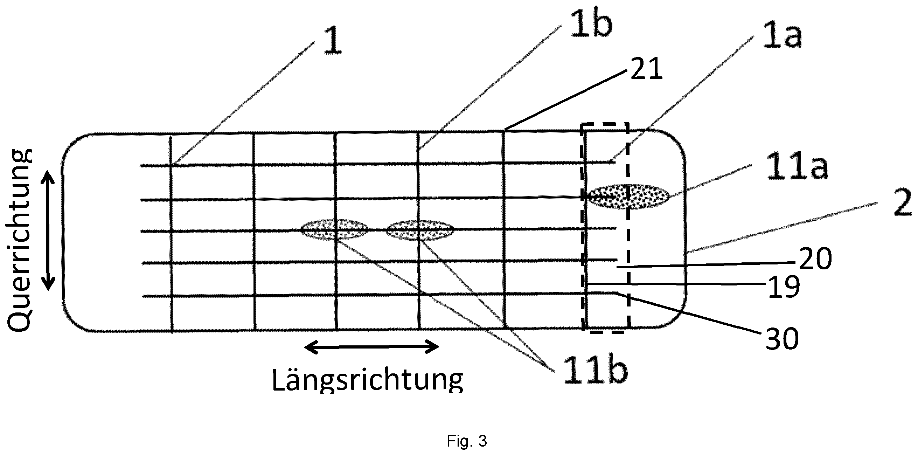

Wie in Fig. 1, Fig. 2, Fig. 6 und Fig. 7 dargestellt, kann die Außenelektrode 1 als Hohlzylinder (aus)gebildet sein und die Außenelektrode 1 kann Querdrähte 1b sowie Längsdrähte 1a aufweisen (vgl. Fig. 3). Die Querdrähte 1b können aus einem leitfähigen Material gebildet sein. Die Längsdrähte 1a können aus einem leitfähigen Material mit einem Drahtdurchmesser, der 10 % bis 15 % dünner als ein Drahtdurchmesser der Querdrähte 1b ist, gebildet sein.

Wie in Fig. 3 dargestellt, können die Querdrähte 1b (d.h. Drähte, welche sich in Querrichtung erstrecken) und die Längsdrähte 1a (d.h. Drähte, welche sich in Längsrichtung erstrecken) das Rechteckmaschen-Gewebe 21 bilden. Ein Abstand benachbarter Querdrähte 1b kann das 4- bis 6-fache des Drahtdurchmessers der Längsdrähte 1a betragen.

Wie ferner in Fig. 3 dargestellt, kann ein Abstand von einem äußersten (z.B. letzten) Querdraht 19, welcher in einem Randabschnitt 20 des Rechteckmaschen-Gewebes 21 in Längsrichtung des Rechteckmaschen-Gewebes 21 angeordnet ist, zu einem jeweiligen freien Ende 30 der Längsdrähte 1a (z.B. das sich über den äußersten (z.B. (letzten) Querdraht 19 in dem Randabschnitt 20 hinauserstreckt) in dem Randabschnitt 20 das 0,8- bis 1,5-fache (, besonders vorteilhaft das 1,0-fache) des Abstands benachbarter (z.B. unmittelbar benachbarter) Querdrähte 1b des Rechteckmaschen-Gewebes 21 betragen. Hierbei können die jeweiligen freien Enden 30 eine messerkantige oder nadelartige Abschnittskante (z.B. Elektrodenkante) aufweisen. Die für ein Ende des Rechteckmaschen-Gewebes 21 dargestellte Konfiguration gilt gleichermaßen für das entgegengesetzte Ende des Rechteckmaschen-Gewebes 21 (in Fig. 3 nicht hervorgehoben, aber im Ergebnis dargestellt). Hierdurch ergibt sich/entsteht am Ende der Längsdrähte 1a ein erster Plasmaaktuator 11a.

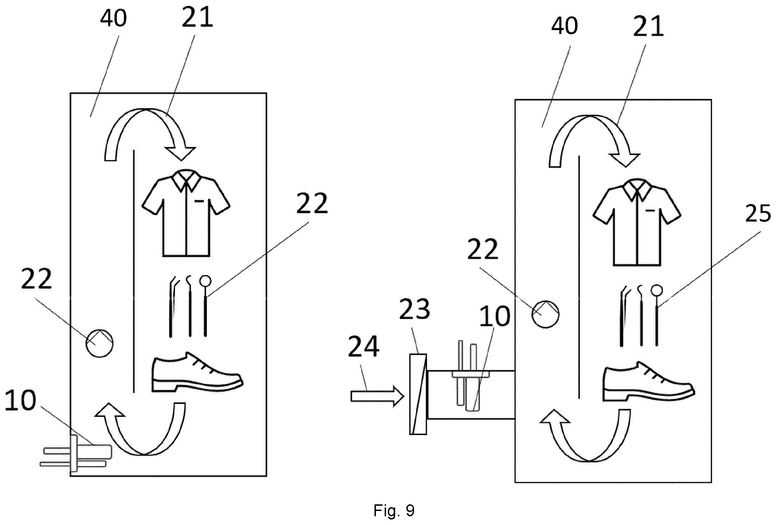

Wie in Fig. 9 dargestellt, stellt ein Aspekt der Erfidung die Verwendung einer in dieser Patentschrift beschriebenen Vorrichtung 10 zur Steckmontage der Vorrichtung 10 mittels der Steckvorrichtung 3 in einer vordefinieren Öffnung an einem Reaktor bereit.

Ferner stellt ein Aspekt der Erfindung ein Verfahren gemäß Anspruch 13 wobei das Verfahren aufweist: Anlegen einer Wechselspannung mit einem Spannungswert und einer Frequenz zwischen der Innenelektrode 4, 4a, 4b (z.B. mittels der Wechselspannung-Anschlussleitung 6) und der Außenelektrode 1 (z.B. mittels der Masse-Anschlussleitung 5) zum Erzeugen einer atmosphärischen Barriereentladung an der Außenelektrode 1; Messen einer UV-Intensität der erzeugten atmosphärischen Barriereentladung (d.h. des erzeugten Plasmas) mittels der Photodiode 9; Steuern des Spannungswerts und der Frequenz basierend auf der gemessenen UV-Intensität, sodass die Vorrichtung 10 zur atmosphärischen Barriereentladung im Bereich eines elektrischen Resonanzzustands (z.B. eines elektrischen Resonanzzustands der Vorrichtung 10 zur atmosphärischen Barriereentladung) betrieben wird.

Ferner kann Verfahren aufweisen: Steuern des Spannungswerts und der Frequenz, sodass in der Vorrichtung 10 zur atmosphärischen Barriereentladung gleichzeitig und räumlich getrennt voneinander ein Ozon-dominiertes Plasma und ein Stickstoff-dominiertes Plasma erzeugt ist/wird.

Ferner kann das Verfahren aufweisen: Betreiben der Vorrichtung 10 zur atmosphärischen Barriereentladung an einem Reaktor (z.B. einem Entkeimungsschrank); Zuführen von gefilterter Luft von außerhalb des Reaktors durch die Vorrichtung 10 zur atmosphärischen Barriereentladung hindurch; Plasma-Behandeln der zugeführten Luft mittels der Vorrichtung 10 zur atmosphärischen Barriereentladung; Zuführen der plasma-behandelten Luft in den Reaktor.

Generell gilt: Eine dielektrische Barriereentladung (DBD) emittiert eine breite Palette von elektromagnetischer Strahlung, einschließlich Ultraviolettstrahlung (UV). Es gibt zwei Arten von UV-Strahlung, die von einer DBD abgegeben werden können: UV-C (Wellenlänge von 100 nm - 280 nm) und UV-Vakuum (Wellenlänge von 10 nm - 200 nm).

Es wurde festgestellt, dass bei einer gleichbleibenden Gaszusammensetzung, einem gleichbleibenden Druck und einer gleichbleibenden Entladungsgeometrie die UVC-Abstrahlung eines SMD-Plasmas linear mit der Entladeleistung ansteigt, bis eine Sättigung erreicht ist/wird.

Die UVC-Abstrahlung eines SMD-Plasmas wird hauptsächlich durch die Entladungsleistung bestimmt. Wenn die Entladeleistung erhöht wird, steigt die UVC-Abstrahlung zunächst linear an. Dies liegt daran, dass bei höheren Entladeleistungen mehr Elektronen und Ionen im Plasma erzeugt werden, die dann zu einer höheren Anzahl von UVC-Photonen führen.

Jedoch gibt es auch eine Sättigungsgrenze, bei der eine weitere Erhöhung der Entladeleistung nicht mehr zu einer signifikanten Steigerung der UVC-Abstrahlung führt. Dies kann aufgrund von verschiedenen Faktoren, wie zum Beispiel einer Selbstabsorption von UVC-Strahlung im Plasma oder einer Sättigung der Vorläuferspezies, die zur Erzeugung von UVC-Photonen beitragen, auftreten.

Veränderungen der Umgebungsbedingungen, wie insbesondere der Luftfeuchte und der Temperatur, verändern die Entladeleistung und somit die Erzeugung von UVC-Photonen. Durch eine Ermittlung/Bestimmung der UVC-Strahlung über die Photodiode 9 kann somit das Plasma in einfacher Weise geregelt und gesteuert sein/werden, insbesondere wird damit die Leistungsdichte des SMD-Bereiches auf 0,1 W/cm² bis 0,2 W/cm² gesteuert/geregelt.

In Anbetracht der chemischen Eigenschaften und der elektrostatischen Physik des Plasmas ist es eine Kernidee der vorliegenden Ausführungsform/Erfindung, eine Vorrichtung zur atmosphärischen Barriereentladung (z.B. ein Plasmamodul) für einen simultanen Betrieb mit mindestens zwei verschiedenen Betriebsmodi zu verwenden, welche günstig sowie ohne Schweißnähte und chemisch bedenkliche Kleber hergestellt sein/werden kann und einfach in bestehende Reaktoren integriert sein/werden kann.

Die Anregungsenergien zur Bildung von mindestens zwei verschiedenen Betriebsmodi sind so gestaltet, dass sie zur Bildung von mindestens zwei verschiedenen chemisch zusammengesetzten Plasmen führen. In bevorzugter Weise ist/wird die Außenelektrode 1 in mindestens zwei Segmente, einen inneren Bereich der Außenelektrode 1 und einen äußeren Bereich der Außenelektrode 1, unterteilt.

Ein innerer Bereich der Außenelektrode 1 kann aus einem Rechteckmaschen-Gewebe 21 mit mindestens zwei unterschiedlichen Drahtstärken (der Längsdrähte 1a und der Querdrähte 1b), welcher den Stickoxid-Modus ausbildet, vorzugsweise RNS produziert und die Erzeugung der RNS großteils mittels einer *"volume dielectric barrier discharge"* (VDBD) durchführt, gebildet sein.

Ein äußerer Bereich ist aus Elektrodenkanten der Außenelektrode 1 gebildet, welcher aufgrund von scharfen Kanten zu einer verbesserten Koronaentladung, einem häufigeren Auftreten von Mikroentladungen und einer höheren Temperatur im Entladungsbereich führt. Die Elektrodenkanten, welche im Ozon-Modus betrieben werden, führen zu einer deutlichen Erhöhung der Energiedichten und der effektiven Entladungslängen der dielektrischen Oberflächenentladung. Diese Entladung an den Abschnittskanten/Elektrodenkanten wird generell als "surface *micro discharge"* (SMD) bezeichnet.

Je höher die Anzahl der Filamente pro Fläche und Zeiteinheit ist, desto größer wird der Leistungsfluss, den dielektrische Materialien aufnehmen, und sie werden heißer. Die Temperatur beeinflusst auch die Ionenproduktion und die Ionenverluste. In einem Bereich von etwa 100 °C können an den scharfen (z.B. scharfkantigen) Abschnittskanten (z.B. Elektrodenkanten) keine negativen und positiven Ionen mehr gemessen werden.

Negative Ionen gelten jedoch auch als desinfizierend. Es hat sich durch Experimente ergeben, dass bei Verwendung der Vorrichtung gemäß der Erfindung/Ausführungsform die Temperatur an den Abschnittskanten auf maximal 80 °C, besonders bevorzugt auf unterhalb von 50 °C, zu begrenzen ist, um ideale Desinfektionsbedingungen zu gewährleisten. Es wurde überraschenderweise festgestellt, dass ein Zusammenhang insofern besteht, dass bei höherer Elektrodentemperatur die Intensität der emittierten UV-C-Strahlung höher ist. Hierbei kann diese Intensität beispielsweise mittels einer Photodiode 9 ermittelt werden und dadurch kann die Temperatur so eingestellt werden, dass eine vorteilhafte Intensität erreicht wird.

Ein vollständiger Übergang zwischen O₃ zu NO_{X} in elektrischen Entladungsströmen tritt bei einer Elektrodentemperatur von etwa 90 °C auf.

Es ist bekannt, dass der Ozon-Modus eine höhere Zündspannung als der Stickoxid-Modus erfordert.

Durch die an den (z.B. scharfkantigen) Abschnittskanten auftretenden Mikroentladungen und die effektiven Entladungslängen an den Abschnittskanten wird jedoch die notwendige Zündspannung verringert und der Ozon-Modus ermöglicht. Es tritt aufgrund von den Mikroentladungen am Dielektrikum 2 bei den Abschnittskanten eine um etwa 20 % höhere Temperatur als im VDBD-Bereich auf, was somit die Zündspannung weiter reduziert.

Diese gitterförmige Außenelektrode 1 ist grundsätzlich dünn und weist an den axial-gerichteten Enden (z.B. den Enden in ihrer Längsrichtung) eine messerkantige oder nadelartige Abschnittskannte auf. Dies führt dazu, dass an den Enden hohe Feldstärken entstehen, an denen Entladungen in einem atmosphärischen Gas, wie zum Beispiel Luft, auftreten. Auf diese Weise entsteht eine kriechentladungsartige Entladung verbunden mit einer Vielzahl von Mikroplasmen. Dieser SMD-Bereich an den Abschnittskanten wird zweckmäßig dahingehend optimiert, dass der Anteil an Ozon möglichst hoch und der Anteil an Stickoxiden möglichst gering ist.

Der maximale Ozongehalt des Plasmas, welches im Ozon-Modus (SMD-Bereich) erzeugt ist/wird, ist generell niedriger als die hygienischen Grenzwerte für Ozon, welche bei 0,1 ppm als Schwellenwert und 0,3 ppm als Höchstgrenze liegen.

Nachteilig zeigt sich die Verwendung eines Siebzylinders als Außenelektrode (z.B. einer Maschen-Außenelektrode) sofern die Kette und der Schuss des Gewebes dieselben Drahtdurchmesser und dieselben Maschenweiten aufweisen. In den Poren des Gewebes sammelt sich vermehrt Schmutz, insbesondere Ammonium-Nitrit und Ammonium-Nitrat, in Form einer weißen Ablagerung, an. Dies sind Nebenprodukte und diese entstehen bevorzugt aus einer Reaktion von Ammoniak und Ozon, welche sich in dem zu behandelnden Gas, insbesondere Umgebungsluft, in geringen Konzentrationen befinden.

Speziell in den Poren des Gewebes, bei gleichen Drahtdurchmessern von Kette und Schuss, sind diese vorzufinden. Der Grund liegt darin, dass sich in den Poren Totzonen befinden und eine Überströmung, und somit eine Abreinigung, nicht ausreichend stattfindet. Die Verwendung von dünnen Drahtdurchmessern, und somit kleineren Totzonen, in der Entladungszone kann diesen Nachteil nicht ausschließen. Begründet wird dies damit, dass bei den in dieser Anmeldung zitierten Dokumenten im optimalen Betrieb, welche kleine Plasmaröhren verwenden, ein Mindestdrahtdurchmesser vorliegen muss, um Gaswirbel in der Entladungszone zu erzeugen.

Gelöst werden kann dieses Problem, indem die Querdrähte 1b (Kette) aus einem leitfähigen Draht mit einem Drahtdurchmesser von 0,25 bis 0,35 mm, vorzugsweise 0,315 mm gebildet sind, und die Längsdrähte 1a (Schuss) aus einem leitfähigen Material, vorzugsweise aus Edelstahl, mit einem Drahtdurchmesser 10 % bis 15 % dünner, besonders bevorzugt 12,5 % dünner als der Drahtdurchmesser der Querdrähte 1b (Kette) gebildet sind.

Des Weiteren können die Abstände der Längsdrähte 1a zueinander (Maschenweite) zwischen 0,30 mm und 0,5 mm, besonders bevorzugt 0,45 mm, sein. Die Abstände zwischen den Querdrähten 1b können das 3-fache bis 4-fache, besonders bevorzugt das 3,2-fache, der Abstände zwischen den Längsdrähten 1a betragen.

Gemäß der Ausführungsform sind die um 10 % bis 15 % dünneren Drähte Längsdrähte 1a in Längsrichtung. Unter Längsrichtung wird verstanden, dass die dünneren Drähte parallel zur Achse (z.B. Längsachse, z.B. Zylinderachse) des hohlzylindrischen Dielektrikums 2 angeordnet sind. Somit kann eine um bis zu 3-fache Anzahl an dünneren Drähten (d.h. Querdrähten 1b), gegenüber der Anzahl an Drähten (d.h. Längsdrähten 1a) in radialer Richtung, in axialer Richtung angeordnet sein.

Unter den Querdrähten 1b werden diejenigen Drähte im Rechteckmaschen-Gewebe 21 verstanden, welche tangential um das hohlzylindrische Dielektrikum 2 geführt sind/werden (z.B. und senkrecht zu den Längsdrähten 1a sind).

Die Elektrodenanordnung (d.h. eine Anordnung von Innenelektrode 4, 4a, 4b und Außenelektrode 1) kann so ausgebildet sein, dass die dielektrische Barriereentladung gleichzeitig mit zwei unterschiedlichen Betriebsmodi hervorgerufen werden kann (z.B. hervorgerufen wird). Dabei kann das Rechteckmaschen-Gewebe 21 so gestaltet sein, dass die zugeführte Anregungsenergie ausreicht, ein Stickstoffspezien (RNS)-haltiges Plasma zu erzeugen und gleichzeitig ein Sauerstoffspezien (ROS/Ozon)-haltiges Plasma zu erzeugen.

Ein Rechteckmaschen-Gewebe 21 kann hierbei ein Gewebe sein, welches aus parallel verlaufenden Längsdrähten 1a und Querdrähten 1b gebildet sein kann, die in rechten Winkeln miteinander verwebt sind. Die Größe der Maschen wird üblicherweise in Maschenweite angegeben, welche den Abstand zwischen den parallelen Drähten in Längsrichtung (d.h. den Längsdrähten 1a) und Querrichtung (d.h. den Querdrähten 1b) des Gewebes beschreibt. Die Maschenweite kann auch durch die Anzahl der Maschen pro Quadratzentimeter oder durch das Gewicht des Gewebes pro Quadratmeter definiert sein/werden.

Dies kann mit zwei verschiedenen Drahtdurchmessern des Rechteckmachen-Gewebes 21 der Außenelektrode 1 (d.h. indem ein Drahtdurchmesser der Längsdrähte 1a des Rechteckmaschen-Gewebes 21 und ein Drahtdurchmesser der Querdrähte 1b des Rechteckmaschen-Gewebes 21 voneinander verschieden sind) erreicht sein/werden. Dabei führen die Längsdrähte 1a mit dem dünneren Drahtdurchmesser, welche axial am hohlzylindrischen Dielektrikum 2 geführt sind, an den Abschnittskanten zu einer höheren elektrischen Feldstärke über dem hohlzylindrischen Dielektrikum 2, was zu einer höheren Leistung und somit zum Ozon-Modus führt.

Die Feldstärke ist aufgrund der Elektrodengeometrie so gewählt, dass sich die Länge der Mikroentladungen vorzugsweise zwischen 1,0 mm bis 3 mm, besonders bevorzugt zwischen 1,8 mm und 2,0 mm an der Oberfläche des hohlzylindrischen Dielektrikums 2 im SMD-Bereich ergeben/entladen.

An den dickeren Drähten im VDBD-Bereich ist die Feldstärke geringer und somit ist die Anregungsenergie geringer als bei den dünneren Drähten der zylinderförmigen Außenelektrode 1 und die Anregungsenergie reicht aus, um ein stickstoffhaltiges Gas zu erzeugen.

Das Rechteckmaschen-Gewebe 21 kann als Elektrode in der DBD eingesetzt sein/werden, um die Entladungszone zu vergrößern und/oder die Gasentladungseffizienz zu erhöhen. Die Kombination aus verschiedenen Drahtdurchmessern kann dazu beitragen, eine gleichmäßigere Entladung im Rechteckmaschen-Gewebe 21 zu erzeugen und das elektrische Feld zu modifizieren, um eine effektivere Entladung zu erreichen.

Die Vorrichtung 10 gemäß der Ausführungsform/Erfindung kann die gleichzeitige Erzeugung von reaktiven Sauerstoffspezies (ROS) und reaktiven Stickstoffspezies (RNS) ermöglichen, wobei eine Erzeugung von Ozonkonzentrationen über 0,3 ppm vermieden werden kann.

Die Elektroden (d.h. die Innenelektrode 4, 4a, 4b und die Außenelektrode 1) können so gestaltet sein, dass die Vorrichtung 10 zur atmosphärischen Barriereentladung (d.h. der Plasmakopf) als Plasmaaktuator funktioniert, welcher durch die Erzeugung eines Plasmafelds eine Strömung, welche Plasmagas aufweist, erzeugt.

Dies ermöglicht einen Betrieb ohne Zwangsbelüftung, d.h. beispielsweise einen Betrieb ohne Verwendung eines (z.B. separaten) Ventilators. Der Betrieb mit Zwangsbelüftung ist auch möglich und verstärkt die Vermischung der Plasmagase, welche im Stickoxid- und im Ozon-Modus simultan erzeugt werden. Durch diese Vermischung werden hochreaktive RNS und ROS, insbesondere N₂O₅ effektiv und stabil erzeugt. N₂O₅ gilt als gut wasserlöslich, was eine Keimreaktion an Oberflächen verstärkt. Oberflächen, insbesondere Gewebe, weisen aufgrund einer Luftfeuchte von 20% bis 60% eine Grundfeuchte auf, in der N₂O₅ besonders gut aufgenommen wird und zur Entkeimung effektiv beiträgt.

Die Vorrichtung 10 gemäß der Ausführungsform/Erfindung kann so ausgebildet sein, dass sie modular und segmentiert aufgebaut ist. Die Steckvorrichtung 3 ist ausgestaltet, dass sie parallel oder, besonders bevorzugt, seriell ausgeführt sein/werden kann (d.h. dass mehrere Steckvorrichtungen 3 parallel oder seriell angeordnet/geschaltet sein/werden können). Mit zunehmender Anzahl von Modulen in Serie (d.h. beispielsweise mit zunehmender Anzahl von getrennten Außenelektroden 1 oder beispielsweise mit zunehmender Anzahl von segmentierten Außenelektroden 1), welche auf mindestens einem Dielektrikum 2 angeordnet sind, kann die gesamte Entladungsleistung an einen Luftstrom angepasst sein/werden.

Mit zunehmender Anzahl segmentierter Elektroden (z.B. Außenelektroden 1) ist/wird der Kanteneffekt (d.h. der Effekt, dass Entladungen beispielsweise an (scharfen) Abschnittskanten bevorzugt auftreten) stärker ausgeprägt und es können viele Strompulse mit geringerer Amplitude erzeugt sein/beobachtet werden. Die signifikante Zunahme der Mikroentladungen bedeutet, dass mehr Entladungskanäle erzeugt sind/werden. Als Hauptursache hierfür gilt die lokal verstärkte Koronaentladung, die durch die Abschnittskante der Elektrode (z.B. Außenelektrode 1) verursacht wird.

Die Vorrichtung gemäß der Ausführungsform/Erfindung ist zur Desinfektion und/oder Geruchsneutralisation von Ausrüstungen, Bekleidungen, insbesondere Schuhen (Stiefeln), technischen oder medizinischen Produkten und Gegenständen, Lebensmitteln, Tieren, insbesondere Bienenstöcke, zur Milbenentfernung in einem geschlossenen System vorgesehen.

Der Plasmaaktuator kann einen Luftstrom, welcher reaktive Sauerstoff- und Stickstoffspezies aufweist, erzeugen, welcher sich beispielsweise in einem geschlossenen System (Reaktionskammer) mit einem Volumen von max. 5 m³ befinden kann.

Für eine vorgegebene Geometrie des Plasmaaktuators und eine vorgegebene Resonanzfrequenz ist die Anregungsenergie von der dielektrischen Barrierespannung abhängig. Diese wird gemäß der Ausführungsform/Erfindung so weit angepasst, dass beginnend an den scharfkantigen Abschnittskanten typische filamentäre Mikroentladungen stattfinden.

Bei weiterer Erhöhung der Spannung bildet sich eine Entladung an den dünneren Drähten des Rechteckmaschen-Gewebes 21 im VDBD-Bereich. Ab einer Stromstärke von etwa 1 mA pro cm² des Rechteckmaschen-Gewebes 21 beginnt eine Entladung bei den dickeren Drähten, und somit eine simultane Entladung im Ozon- und im Stickoxid-Modus. Nach einer Einlauffase von etwa 5 Minuten können sich die optimalen Temperaturen im Plasmaaktuator einstellen. Diese optimalen Temperaturen sind bevorzugt von 20 °C bis 60 °C, weiter bevorzugt von 25 °C bis 60 °C, besonders bevorzugt von 40 °C bis 50 °C im Stickoxid-Modus (VDBD-Bereich) und von 40 °C bis 70 °C, besonders bevorzugt von 45 °C bis 60 °C, besonders bevorzugt von 45 °C bis 50 °C im Ozon-Modus (SMD-Bereich).

Es zeigte sich, dass ab einer Temperatur von 50 °C an den Abschnittskanten Ozonkonzentrationen unter 0,3 ppm gebildet werden können.

Es versteht sich auch, dass die Leistung einer Plasmaeinheit mit dielektrischer Barriereentladung von der Anzahl der Segmente/Module abhängt.

Die Vorrichtung 10 gemäß der Ausführungsform/Erfindung kann an der Außenelektrode 1 ein Verhältnis eines Durchmessers des hohlzylindrischen Dielektrikums 2 zur Elektrodenlänge der Außenelektrode 1 von 1 bis 2, vorzugsweise von 1 bis 1,8, besonders bevorzugt von 1,2 bis 1,3 aufweisen.

Die Vorrichtung 10 gemäß der Ausführungsform/Erfindung kann ein Verhältnis einer Länge der Außenelektrode 1 zur Entladungsbreite im SMD-Bereich bevorzugt von 3 bis 10, besonders bevorzugt von 4 bis 8, am meisten bevorzugt von 4,4 bis 4,6 aufweisen. Hierunter wird verstanden, dass an den Abschnittskanten, bei einer vorgegebenen Elektrodenkonfiguration und einer Ausführung des Dielektrikums 2 gemäß der Ausführungsform/Erfindung, mit einer Dielektrizitätskonstante von 4,6 und einer Wandstärke s von 0,8 m, eine Entladungsbreite an den Abschnittskanten von 1,8 mm bis 2 mm beobachtet werden kann.

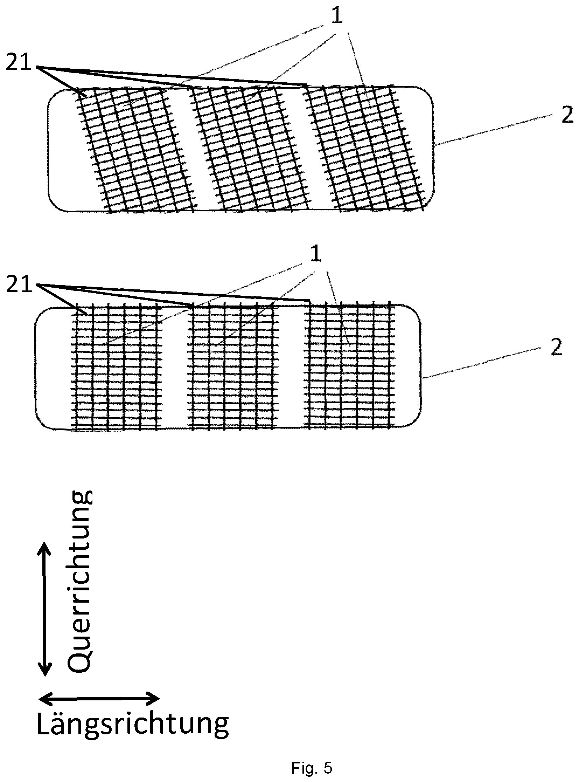

Bei Verwendung von größeren Durchmessern des Dielektrikums 2 kann gemäß einer Ausführungsform die Außenelektrode 1 auf dem Dielektrikum 2 spiralförmig gewickelt (siehe Fig. 5 oben) und/oder modulweise in Segmenten angeordnet (siehe Fig. 5 unten) sein/werden.

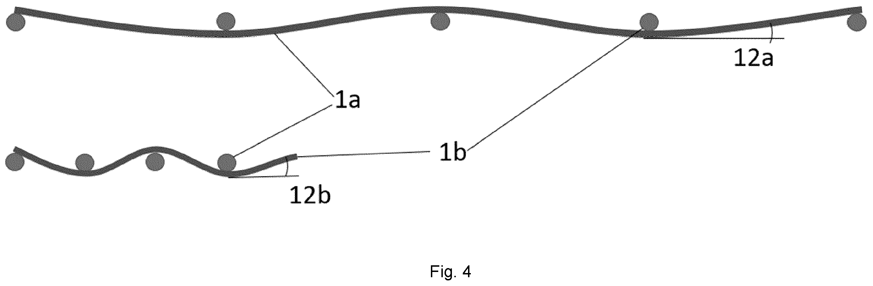

Unter Bezugnahme auf Fig. 4 ist dargestellt, dass gemäß einer Ausführungsform die Drähte (z.B. die Längsdrähte 1a und die Querdrähte 1b) im Rechteckmaschen-Gewebe 21 verschiedene Steigungen 12a, 12b zum Dielektrikum 2 aufweisen. Je größer die Abstände (Maschenweite) der Querdrähte 1b zueinander, desto geringer die Winkel 12a der Längsdrähte 1a zum Dielektrikum 2. Somit kann an den Längsdrähten 1a ein zweiter Plasmaaktuator generiert werden, was durch eine streifenförmige Entladung 11b (siehe Fig. 3) festgestellt werden kann. Generell ist bekannt, dass beim einem Plasmaaktuator die Elektrodenkanten oder Drähte keinen oder nur einen sehr geringen Winkel gegenüber dem Dielektrikum 2 ausbilden. Bevorzugt kann deshalb der Abstand der Querdrähte 1b das 4- bis 6fache, besonders bevorzugt das 5-fache, eines Drahtdurchmessers des Längsdrahtes 1a betragen. Somit ist eine Funktion des Plasmakopfes als Plasmaaktuator gegeben.

Die Länge des Plasmas in einer "Surface Microdischarge" (SMD) an den Abschnittskanten beeinflusst die Ozonproduktion und wird durch die Dielektrizitätskonstante beeinflusst. Die Dielektrizitätskonstante ist ein Maß dafür, wie leicht ein Material elektrische Feldlinien durchdringen lässt. In einem SMD wird ein elektrisches Feld zwischen zwei Elektroden erzeugt, die durch eine dünne Schicht aus Dielektrikum voneinander getrennt sind. Das Dielektrikum beeinflusst die Verteilung des elektrischen Feldes und somit auch die Länge des Plasmas von den Abschnittskanten (her).

Ein höherer Wert der Dielektrizitätskonstante des Dielektrikums führt in der Regel zu einer stärkeren Abschwächung des elektrischen Feldes und somit zu einer kürzeren Plasmalänge. Ein niedrigerer Wert der Dielektrizitätskonstante hingegen führt zu einer geringeren Abschwächung des elektrischen Feldes und somit zu einer längeren Plasmalänge. Für die Vorrichtung gemäß der Ausführungsform/Erfindung wird vorzugsweise ein Material für das Dielektrikum mit hoher Durchschlagsfestigkeit und geringer Verlustleistung bevorzugt, um eine effektive Isolation zwischen den Elektroden (d.h. der Innenelektrode 4, 4a, 4b und der Außenelektrode 1) zu gewährleisten und eine hohe Leistungsdichte zu erreichen.

Bevorzugt wird ein Material mit einem dielektrischen Verlustfaktor (tan φ) von 10*10⁻⁴ bis 30*10⁻⁴ verwendet.

Vorzugsweise kann die Glasröhre (d.h. das hohlzylindrische Dielektrikum 2) aus Borosilikatglas ausgebildet sein. Die Verwendung von Borosilikatglas, mit einer Wandstärke von 0,8 mm, dessen Dielektrizitätszahl ε etwa zwischen 4,3 und 5,5, bevorzugt ε = 4,9, ist, trägt zu einer Entladung bei, bei der besonders im Ozon-Modus (SMD-Bereich) die Temperaturentwicklung und die Länge der Entladungen vorteilhaft ist. In der Vorrichtung gemäß der Ausführungsform/Erfindung kann eine Dielektrizitätskonstante von ε = 4,9 und ein dielektrischer Verlustfaktor (tan φ) von 22*10⁻⁴ besonders bevorzugt sein.

Bevorzugt kann eine gesamte Leistungsdichte von 0,5 W/cm² bis 2,2 W/cm² besonders bevorzugt 1,5 W/cm², an der gesamten Entladungsfläche der Außenelektrode 1 sein. Dadurch ergibt sich im Ozon-Modus (SMD-Bereich) eine Leistungsdichte von 0,1 W/cm² bis 0,2 W/cm².

Generell gilt, dass bei einer dielektrischen Barriereentladung zum Erreichen einer Zündspannung eine hohe Mindest-Elektronendichte erforderlich ist. Diese erforderliche Mindest-Elektronendichte ist bei einer dielektrischen Barriereentladung (DBE) durch eine Ladungsträgeranlagerung am Dielektrikum 2 und eine Restleitfähigkeit der ionisierten Luft im Gasraum der Außenelektrode 1 möglich. Diese Charakteristik der DBE ist auch unter dem Memoryeffekt bekannt. Je höher die angelegte Frequenz, desto ausgeprägter der Memoryeffekt. Eine hohe Dielektrizitätskonstante in Kombination mit einer Frequenz im kHz-Bereich führt aufgrund des Memoryeffektes zu verstärkt auftretenden Filamentbildungen und zu einer schwachen Ausprägung des SMD-Bereichs.

Mit einem lonometer IM 806 der Firma Umweltanalytik Holbach GmbH in D-66687 Wadem lässt sich beispielsweise die Luftionenkonzentration von positiv und negativ geladenen Sauerstoff-Ionen kontinuierlich messen. Der Messbereich beträgt 0 bis 40 Millionen Ionen/cm³. In der Ausführungsform wird beispielsweise eine negative Ionenkonzentration von 1,1 Millionen negativen Ionen pro cm³ - bei atmosphärischen Bedingungen wie 47,30 % relative Luftfeuchte, 942 hPa und 20 °C - im Abstand von 30 cm zum Plasmakopf festgestellt.

Generell gilt, dass Plasma, welches im Hochfrequenz-Niederspannungszustand erzeugt/betrieben wird, eine gleiche antimikrobielle Aktivität wie Plasma hat, welches im Niederfrequenz-Hochspannungszustand erzeugt/betrieben wird, wenn die Entladungsleistungsdichte gleich ist. Die Vorrichtung 10 gemäß der Ausführungsform/Erfindung kann mit einer Frequenz außerhalb des menschlichen Hörvermögens in der Resonanzfrequenz betrieben werden. Bevorzugt werden Frequenzen von beispielsweise 17 kHz bis 50 kHz, je nach der Kapazität der Vorrichtung gemäß der Ausführungsform/Erfindung. Besonders bevorzugt sind beispielsweise Frequenzen von 20 kHz bis 35 kHz. Bei diesen Frequenzen können aufgrund des Memoryeffektes Mikroentladungen im Bereich von bevorzugt 1,8 mm bis 2,0 mm erzeugt/festgestellt werden.

Es wurde festgestellt, dass die Dielektrika (z.B. das hohlzylindrische Dielektrikum 2) und ihre Eigenschaften eine entscheidende Rolle bei der Bildung von Plasma spielen. Eine dieser Eigenschaften ist die Art des dielektrischen Materials, die zweite dieser Eigenschaften ist die Dicke der dielektrischen Barriere und die dritte dieser Eigenschaften ist die Oberflächenrauhigkeit der dielektrischen Barriere.

Die Rauhigkeit der Oberfläche hat einen großen Einfluss auf die Erzeugung von Plasma. Je rauer die dielektrische Oberfläche ist, desto mehr Elektronen kann sie halten und desto mehr Keimelektronen kann sie für die nächste AC-Halbzyklusentladung der DBD bereitstellen. Die Oberflächenrauhigkeit des dielektrischen Materials reduziert das elektrische Durchschlagsfeld aufgrund seiner unebenen Oberfläche.

Bevorzugt wird beispielsweise eine Rauheit der Fläche unter der Außenelektrode 1 von Ra 1,5 µm bis 10 µm, bevorzugter von 2 µm bis 8 µm µm, am meisten bevorzugt von 3 µm bis 5 µm. Damit ist ausschließlich die Rauhigkeit unter der Außenelektrode 1 gemeint. Beispielsweise sollten die Flächen im SMD-Bereich glatt sein, um die Länge der Mikroentladungen zu verstärken. Beispielsweise ist/wird die Rauhigkeit bevorzugt durch Sandstrahlen hergestellt.

Beispielsweise kann die Vorrichtung 10 gemäß der Ausführungsform/Erfindung mit einer 12 V-Niederspannungsversorgung betrieben sein/werden. Dabei kann die erforderliche Energiemenge aus Sonnen- und Tageslicht erzeugt werden. Dies ist insbesondere vorteilhaft, wenn kleine mobile Anlagen in der freien Natur betrieben werden und kein (stationäres) Stromnetz vorhanden ist. Die 12 V-Niederspannung wird dann über eine elektronische Regelung der Betriebsspannung von 600 Vrms bis 1,3 kVrms erzeugt.

Bevorzugterweise kann der Energieeintrag bei Verwendung der Vorrichtung 10 gemäß der Ausführungsform/Erfindung weniger als 1 Watt pro m³ (W/m³) in geschlossenen Behandlungsvolumina, vorzugsweise weniger als etwa 0,8 W/m³, betragen. Berechnet wird dieser Energieeintrag, indem der für die Ionisation der Luft gemessene Energieaufwand durch das Volumen des Reaktorsystems, in das die Luft eingebracht wird, dividiert wird.

Bevorzugterweise kann der Ionisationsgrad der Luft weniger als 90 Vol.-%, weiter bevorzugt weniger als 80 Vol.-%, insbesondere weniger als 70 Vol.-%, noch weiter bevorzugt weniger als 60 Vol.-%, noch weiter bevorzugt weniger als 50 Vol.-%, weiter bevorzugt weniger als 40 Vol.- %, insbesondere weniger als 30 Vol.-%, und am meisten bevorzugt weniger als 20 Vol.-% betragen. Der Ionisationsgrad wie hierin angegeben bezieht sich nach einer Ausführungsform nicht auf den vorhandenen (gesamten) Sauerstoff, sondern auf den Ozonschwellwert.

Im Rahmen der Erfindung wurde festgestellt, dass sich bei Betriebstemperaturen des Plasmakopfes (z.B. der Vorrichtung 10), nach mindestens fünf Minuten Betrieb, der Ionisationsgrad linear zur Leistungsdichte an der Außenelektrode 1 verhält. Dies kann jedoch nur bei Betriebstemperaturen unterhalb von 60 °C festgestellt werden und ist somit bei atmosphärischen Bedingungen, wie sie in der Reaktionskammer vorkommen, ein Maß für die Intensivität des Plasmas. Laut ATEX-Leitlinien werden unter atmosphärischen Bedingungen in der Regel Umgebungstemperatur von -20 °C bis 60 °C und ein Druckbereich zwischen 0,8 bar (80.000 Pa) und 1,1 bar (110.000 Pa) verstanden.

Im Rahmen der Erfindung wurde unerwarteterweise festgestellt, dass es nach einer Ausführungsform vorteilhaft ist, den Ionisationsgrad der Luft niedrig zu wählen, und gegebenenfalls eine längere Behandlungszeit des zu behandelnden Volumens, mit der schwach ionisierten Luft durchzuführen, um stark oxidierende Bedingungen in dem Medium zu vermeiden. Dementsprechend ist auch die Betriebstemperatur zu wählen.

Beispielsweise kann die Behandlung des Luftvolumens zusätzlich bevorzugt mit dem Plasmaaktuator 10 in einem geschlossenen System 40 in einem kontinuierlichen Verfahren erfolgen. Das erzeugte Plasmagas, welches RNS und ROS aufweist, wird anschließend auf die zu behandelnden Objekte (z.B. Gegenstände) 25 geleitet/geführt (siehe Fig. 9).

Eine ständige Luftumwälzung 21 kann beispielsweise mit einem Ventilator 22 ermöglicht sein/werden. Bevorzugt wird die Luft der ständigen Umwälzung behandelt, wobei der Luftstrom nicht zwingend direkt auf den Plasmaaktuator 10 geleitet wird. Die Vorrichtung 10 gemäß der Ausführungsform/Erfindung ermöglicht aufgrund der Elektrodengeometrie eine eigenständige Luftströmung. Beispielsweise wird gefilterte Luft besonders bevorzugt von außerhalb des Reaktors über eine Strömung 24 zum Plasmaaktuator 10 geleitet. Dies reduziert eine mögliche Verschmutzung des Plasmaaktuators (z.B. der Vorrichtung 10) durch Staub und Schmutzpartikel in der Zirkulation. Zur Luftfilterung werden besonders bevorzugt Staubfilter 23 der ISO Klassen G1 bis G3 verwendet.

Nach einer bevorzugten Ausführungsform liegt die kontinuierliche Behandlungszeit zur Entkeimung zwischen 1 Minute und 120 Minuten, weiter bevorzugt zwischen 20 Minuten und 60 Minuten, besonders bevorzugt zwischen 25 Minuten und 35 Minuten.

Die Behandlung der mit RNS und ROS angereicherten Umwälzluft kann so durchgeführt werden, dass eine Inaktivierung (Log-Reduktion) der Konzentrationen von Mikroorganismen und Viren an den zu behandelnden Oberflächen mindestens um log 3, weiter bevorzugt um log 4, noch weiter bevorzugt um log 5 und am meisten bevorzugt um log 6 geschieht. Gemessen wird hierbei die Keimzahl der "unbehandelten" Oberfläche, das heißt vor der Behandlung mit ROS und RNS. Dies ergibt den Ausgangswert. Dieser Ausgangswert wird mit dem Endwert verglichen, der nach dem Beenden der Plasmabehandlung gemessen wird.

Beispielsweise kann es zusätzlich bevorzugt sein, dass im Verfahren gemäß der Ausführungsform lediglich Plasmagas, aber keine zusätzlichen Oxidationsmittel, wie zum Beispiel Chlor und Chlorgas, Wasserstoffperoxid oder ähnliches, eingesetzt werden.

### Zitate

[1] M. J. Pavlovich, "Antimicrobial Applications of Ambient-Air Plasmas," dissertation, University of California, Berkeley, 2014.
[2] W. X. e. al, "Mode transition of air surface micro-discharge and its effect on the water activation and antibacterial activity," Plasma Sources Science and Technology, Bd. Volume 29, Nr. 5, 2022.
[3] Z. W. e. al, "Combination of NOx mode and O3 mode air discharges for water activation to produce a potent disinfectant," Plasma Sources Science and Technology, Bd. Volume 31, Nr. 5, 2022.

## Patentansprüche

1. Vorrichtung (10) zur atmosphärischen Barriereentladung, welche aufweist:
mindestens ein hohlzylindrisches Dielektrikum (2),
welches eine innere Mantelfläche (2a) sowie eine äußere Mantelfläche (2b) hat und
welches eine Dielektrizitätskonstante größer 4 hat,
eine Steckvorrichtung (3), welche mindestens ein Haltemittel (3a, 3b, 3c) aufweist,
an welchem mindestens eine Innenelektrode (4, 4a, 4b) und mindestens eine Außenelektrode (1) angeordnet sind und
an welchem das hohlzylindrische Dielektrikum (2) angeordnet ist, um an der Steckvorrichtung (3) gehalten zu sein,
die Innenelektrode (4, 4a, 4b), welche innerhalb des hohlzylindrischen Dielektrikums (2) angeordnet ist und an der inneren Mantelfläche (2a) anliegt, und
eine Außenelektrode (1), welche ein Rechteckmaschen-Gewebe (21) aufweist und welche an der äußeren Mantelfläche (2b) des Dielektrikums (2) anliegt,
wobei die Steckvorrichtung (3) eine plattenförmige Basis (11) aufweist, an welcher das mindestens eine Haltemittel (3a, 3b, 3c) ausgebildet ist,
wobei
das mindestens eine Haltemittel (3a, 3b, 3c) ein erstes Halteelement (3a, 3b) aufweist, welches von der ersten Fläche (13) der plattenförmigen Basis (11) her vorsteht, und
das erste Haltemittel (3a, 3b) dazu eingerichtet ist, die Innenelektrode (4, 4a, 4b) mit einem Anpressdruck zwischen 10 N/cm² und 50 N/cm² gegen das hohlzylindrische Dielektrikum (2) zu drücken,
wobei das erste Halteelement (3a, 3b) ein Spannstift ist,
wobei die Innenelektrode (4, 4b) aus einem Maschengewebe aus einem paramagnetischen Material gebildet ist, und
wobei
der Spannstift (3b) einen Längsschlitz (14) aufweist,
die Innenelektrode (4, 4b) um den Spannstift (3b) herum gewickelt ist, um an einer äußeren Mantelfläche (15) des Spannstifts (3b) anzuliegen,
zwei entgegengesetzte Endabschnitte (16, 17) der Innenelektrode (4, 4b) in den Längsschlitz (14) eingeführt sind, und
das hohlzylindrische Dielektrikum (2) um den Spannstift (3b) herum so angeordnet ist, dass es die Innenelektrode (4, 4b) gegen die äußere Mantelfläche (15) des Spannstifts (3b) drückt.

2. Vorrichtung (10) gemäß Anspruch 1, wobei die Steckvorrichtung (3) ferner einen Permanentmagneten (8) aufweist, welcher koaxial zur Innenelektrode (4, 4a, 4b) angeordnet ist.

3. Vorrichtung (10) gemäß Anspruch 1 oder 2, wobei
die plattenförmige Basis (11) ferner Montageelemente (3d) aufweist, welche optional an einem Rand der plattenförmigen Basis (11) angeordnet sind.

4. Vorrichtung (10) gemäß irgendeinem der Ansprüche 1 bis 3, wobei
die plattenförmige Basis (11) ein Photodiode-Halteelement (12) aufweist, welches von einer ersten Fläche (13) der plattenförmigen Basis (11) her vorsteht, und
die Steckvorrichtung (3) ferner eine Photodiode (9) zur Überwachung der atmosphärischen Barriereentladung aufweist, welche an dem Photodiode-Halteelement (12) angeordnet ist und welche nach außen hin exponiert ist.

5. Vorrichtung (10) gemäß irgendeinem der Ansprüche 1 bis 3, wobei
die plattenförmige Basis (11) ein UV-C-emittierende-Vorrichtung-Halteelement (12) aufweist, welches von einer ersten Fläche (13) der plattenförmigen Basis (11) her vorsteht, und
die Steckvorrichtung (3) ferner eine UV-C-emittierende Vorrichtung (9) aufweist, welche an dem UV-C-emittierende-Vorrichtung-Halteelement (12) angeordnet ist und welche nach außen hin exponiert ist.

6. Vorrichtung (10) gemäß irgendeinem der Ansprüche 1 bis 5, wobei die Innenelektrode (4, 4a) aus einem temperaturbeständigen leitfähigen Elastomer gebildet ist.

7. Vorrichtung (10) gemäß Anspruch 6, wobei die Innenelektrode (4, 4a) mittels des ersten Halteelements (3a) gegen die innere Mantelfläche (2a) gedrückt ist.

8. Vorrichtung (10) gemäß irgendeinem der Ansprüche 1 bis 7, wobei
das erste Halteelement (3a, 3b) ein Durchgangsloch (18) hat,
die Vorrichtung (10) eine Wechselspannung-Anschlussleitung (6) aufweist, welche mit der Innenelektrode (4, 4a, 4b) elektrisch verbunden ist, und
die Wechselspannung-Anschlussleitung (6) durch das Durchgangsloch (18) des ersten Halteelements (3a, 3b) hindurch geführt ist.

9. Vorrichtung (10) gemäß irgendeinem der Ansprüche 1 bis 8, wobei
die Vorrichtung (10) eine Masse-Anschlussleitung (5) aufweist, welche mit der Außenelektrode (1) elektrisch verbunden ist,
das mindestens eine Haltemittel (3a, 3b, 3c) ein zweites Halteelement (3c) aufweist, welches von der ersten Fläche (13) der plattenförmigen Basis (11) her vorsteht, und
das zweite Haltemittel (3c) dazu eingerichtet ist, die Masse-Anschlussleitung (5) mechanisch zu führen.

10. Vorrichtung (10) gemäß irgendeinem der Ansprüche 1 bis 9, wobei
die Außenelektrode (1) als Hohlzylinder gebildet ist und Querdrähte (1b) sowie Längsdrähte (1a) aufweist,
die Querdrähte (1b) aus einem leitfähigen Material gebildet sind, und
die Längsdrähte (1a) aus einem leitfähigen Material mit einem Drahtdurchmesser, der 10% bis 15% dünner als ein Drahtdurchmesser der Querdrähte (1b) ist, gebildet sind.

11. Vorrichtung (10) gemäß Anspruch 10, wobei
die Querdrähte (1b) und die Längsdrähte (1a) das Rechteckmaschen-Gewebe (21) bilden, und
ein Abstand benachbarter Querdrähte (1b) das 4- bis 6-fache des Drahtdurchmessers der Längsdrähte (1a) beträgt,
wobei optional ein Abstand von einem äußersten Querdraht (19), welcher in einem Randabschnitt (20) des Rechteckmaschen-Gewebes (21) in Längsrichtung des Rechteckmaschen-Gewebes (21) angeordnet ist, zu einem jeweiligen freien Ende (30) der Längsdrähte (1a) in dem Randabschnitt (20) das 0,8- bis 1,5-fache des Abstands benachbarter Querdrähte (1b) des Rechteckmaschen-Gewebes (21) beträgt.

12. Verwendung einer Vorrichtung (10) gemäß irgendeinem der vorherigen Ansprüche zur Steckmontage der Vorrichtung (10) mittels der Steckvorrichtung (3) in einer vordefinieren Öffnung an einem Reaktor.

13. Verfahren zum Betreiben einer Vorrichtung (10) zur atmosphärischen Barriereentladung gemäß irgendeinem der Ansprüche 1 bis 11 sofern in Kombination mit Anspruch 4, wobei das Verfahren aufweist:
Anlegen einer Wechselspannung mit einem Spannungswert und einer Frequenz zwischen der Innenelektrode (4, 4a, 4b) und der Außenelektrode (1) zum Erzeugen einer atmosphärischen Barriereentladung an der Außenelektrode (1);
Messen einer UV-Intensität der erzeugten atmosphärischen Barriereentladung mittels der Photodiode (9);
Steuern des Spannungswerts und der Frequenz basierend auf der gemessenen UV-Intensität, sodass die Vorrichtung (10) zur atmosphärischen Barriereentladung im Bereich eines elektrischen Resonanzzustands betrieben wird.

14. Verfahren gemäß Anspruch 13, welches ferner aufweist:
Steuern des Spannungswerts und der Frequenz, sodass in der Vorrichtung (10) zur atmosphärischen Barriereentladung gleichzeitig und räumlich getrennt voneinander ein Ozon-dominiertes Plasma und ein Stickstoff-dominiertes Plasma erzeugt wird.

15. Verfahren gemäß Anspruch 13 oder 14, welches ferner aufweist:
Betreiben der Vorrichtung (10) zur atmosphärischen Barriereentladung an einem Reaktor **(z.B.** einem Entkeimungsschrank);
Zuführen von gefilterter Luft von außerhalb des Reaktors durch die Vorrichtung (10) zur atmosphärischen Barriereentladung hindurch;
Plasma-Behandeln der zugeführten Luft mittels der Vorrichtung (10) zur atmosphärischen Barriereentladung;
Zuführen der plasma-behandelten Luft in den Reaktor.

## Claims

1. Device (10) for atmospheric barrier discharge, comprising:
at least one hollow cylindrical dielectric (2),
which has an inner jacket surface (2a) as well as an outer jacket surface (2b), and
which has a dielectric constant greater than 4,
a plug-in device (3) which comprises at least one holder (3a, 3b, 3c),
on which at least one inner electrode (4, 4a, 4b) and at least one outer electrode (1) are arranged, and
on which the hollow cylindrical dielectric (2) is arranged in order to be held on the plug-in device (3),
the inner electrode (4, 4a, 4b), which is arranged inside the hollow cylindrical dielectric (2) and abuts against the inner jacket surface (2a), and
an outer electrode (1) which comprises a rectangular mesh fabric (21) and which abuts against the outer jacket surface (2b) of the dielectric (2),
wherein the plug-in device (3) comprises a plate-shaped base (11) on which the at least one holder (3a, 3b, 3c) is formed,
wherein
the at least one holder (3a, 3b, 3c) comprises a first holding element (3a, 3b) which protrudes from the first surface (13) of the plate-shaped base (11), and
the first holder (3a, 3b) is configured to push the inner electrode (4, 4a, 4b) against the hollow cylindrical dielectric (2) with a contact pressure between 10 N/cm² and 50 N/cm², wherein the first holding element (3a, 3b) is a spring pin,
wherein the inner electrode (4, 4b) is formed from a mesh fabric made of a paramagnetic material, and
wherein
the spring pin (3b) comprises a longitudinal slit (14),
the inner electrode (4, 4b) is wound around the spring pin (3b) so as to abut against an outer jacket surface (15) of the spring pin (3b),
two opposite end portions (16, 17) of the inner electrode (4, 4b) are inserted into the longitudinal slit (14), and
the hollow cylindrical dielectric (2) is arranged around the spring pin (3b) such that it pushes the inner electrode (4, 4b) against the outer jacket surface (15) of the spring pin (3b).

2. Device (10) according to claim 1, wherein the plug-in device (3) further comprises a permanent magnet (8) which is arranged coaxially with the inner electrode (4, 4a, 4b).

3. Device (10) according to claim 1 or 2, wherein
the plate-shaped base (11) further comprises mounting elements (3d) which are optionally arranged at an edge of the plate-shaped base (11).

4. Device (10) according to any one of claims 1 to 3, wherein
the plate-shaped base (11) comprises a photodiode holding element (12) which protrudes from a first surface (13) of the plate-shaped base (11), and
the plug-in device (3) further comprises a photodiode (9) for monitoring the atmospheric barrier discharge, which is arranged on the photodiode holding element (12) and which is exposed to the outside.

5. Device (10) according to any one of claims 1 to 3, wherein
the plate-shaped base (11) comprises a UV-C-emitting device holding element (12) which protrudes from a first surface (13) of the plate-shaped base (11), and
the plug-in device (3) further comprises a UV-C-emitting device (9) which is arranged on the UV-C-emitting device holding element (12) and which is exposed to the outside.

6. Device (10) according to any one of claims 1 to 5, wherein the inner electrode (4, 4a) is formed from a temperature-resistant conductive elastomer.

7. Device (10) according to claim 6, wherein the inner electrode (4, 4a) is pushed against the inner jacket surface (2a) by means of the first holding element (3a).

8. Device (10) according to any one of claims 1 to 7, wherein
the first holding element (3a, 3b) has a through hole (18),
the device (10) has an alternating voltage connection line (6) which is electrically connected to the inner electrode (4, 4a, 4b), and
the alternating voltage connection line (6) is guided through the through hole (18) of the first holding element (3a, 3b).

9. Device (10) according to any of claims 1 to 8, wherein
the device (10) comprises a ground connection line (5) which is electrically connected to the outer electrode (1),
the at least one holder (3a, 3b, 3c) comprises a second holding element (3c) which projects from the first surface (13) of the plate-shaped base (11), and
the second holder (3c) is configured to mechanically guide the ground connection line (5).

10. Device (10) according to any one of claims 1 to 9, wherein
the outer electrode (1) is formed as a hollow cylinder and comprises transverse wires (1b) and longitudinal wires (1a),
the transverse wires (1b) are formed from a conductive material, and
the longitudinal wires (1a) are formed from a conductive material with a wire diameter that is 10% to 15% thinner than a wire diameter of the transverse wires (1b).

11. Device (10) according to claim 10, wherein
the transverse wires (1b) and the longitudinal wires (1a) form the rectangular mesh fabric (21), and
a distance of adjacent transverse wires (1b) is 4 to 6 times the wire diameter of the longitudinal wires (1a),
wherein, optionally, a distance from an outermost transverse wire (19), which is arranged in an edge portion (20) of the rectangular mesh fabric (21) in the longitudinal direction of the rectangular mesh fabric (21), to a respective free end (30) of the longitudinal wires (1a) in the edge portion (20) is 0.8 to 1.5 times the distance of adjacent transverse wires (1b) of the rectangular mesh fabric (21).

12. Use of a device (10) according to any one of the previous claims for plug-in mounting of the device (10) by means of the plug-in device (3) in a predefined opening on a reactor.

13. Method for operating a device (10) for atmospheric barrier discharge according to any one of claims 1 to 11 provided that in combination with claim 4, wherein the method comprises:
applying an alternating voltage with a voltage value and a frequency between the inner electrode (4, 4a, 4b) and the outer electrode (1) for generating an atmospheric barrier discharge at the outer electrode (1);
measuring a UV intensity of the generated atmospheric barrier discharge by means of the photodiode (9);
controlling the voltage value and the frequency based on the measured UV intensity so that the device (10) for atmospheric barrier discharge is operated in the region of an electrical resonance state.

14. Method according to claim 13, which further comprises:
controlling the voltage value and the frequency so that, in the device (10) for atmospheric barrier discharge, an ozonedominated plasma and a nitrogen-dominated plasma are generated simultaneously and spatially separated from each other.

15. Method according to claim 13 or 14, which further comprises:
operating the device (10) for atmospheric barrier discharge on a reactor (e.g., a disinfection cabinet);
supplying filtered air from outside the reactor through the device (10) for atmospheric barrier discharge;
plasma treating the supplied air by means of the device (10) for atmospheric barrier discharge;
supplying the plasma-treated air into the reactor.

## Revendications

1. Dispositif (10) pour décharge barrière atmosphérique, comprenant:
au moins un diélectrique cylindrique creux (2),
qui a une surface d'enveloppe intérieure (2a) ainsi qu'une surface d'enveloppe extérieure (2b) et
qui a une constante diélectrique supérieure à 4,
un dispositif d'enfichage (3) qui comprend au moins un moyen de retenue (3a, 3b, 3c),
auquel sont disposées au moins une électrode intérieure (4, 4a, 4b) et au moins une électrode extérieure (1) et
auquel est disposé le diélectrique cylindrique creux (2) afin d'être retenu au dispositif d'enfichage (3),
l'électrode intérieure (4, 4a, 4b) qui est disposée à l'intérieur du diélectrique cylindrique creux (2) et qui vient en butée contre la surface d'enveloppe intérieure (2a), et
une électrode extérieure (1) qui comprend un tissu à mailles rectangulaires (21) et qui vient en butée contre la surface enveloppe extérieure (2b) du diélectrique (2),
le dispositif d'enfichage (3) comprenant une base en forme de plaque (11) à laquelle est formé ledit au moins un moyen de retenue (3a, 3b, 3c),
dans lequel
ledit au moins un moyen de retenue (3a, 3b, 3c) comprend un premier élément de retenue (3a, 3b) qui fait saillie à partir de la première surface (13) de la base en forme de plaque (11), et
le premier moyen de retenue (3a, 3b) est configuré pour presser l'électrode intérieure (4, 4a, 4b) contre le diélectrique cylindrique creux (2) avec une pression de contact comprise entre 10 N/cm² et 50 N/cm²,
dans lequel le premier élément de retenue (3a, 3b) est une goupille élastique,
dans lequel l'électrode intérieure (4, 4b) est formée d'un tissu maillé en un matériau paramagnétique, et
dans lequel
la goupille élastique (3b) comprend une fente longitudinale (14),
l'électrode intérieure (4, 4b) est enroulée autour de la goupille élastique (3b) afin de venir en butée contre une surface d'enveloppe extérieure (15) de la goupille élastique (3b),
deux parties d'extrémité opposées (16, 17) de l'électrode intérieure (4, 4b) sont insérées dans la fente longitudinale (14), et
le diélectrique cylindrique creux (2) est disposé autour de la goupille élastique (3b) de manière à presser l'électrode intérieure (4, 4b) contre la surface d'enveloppe extérieure (15) de la goupille élastique (3b).

2. Dispositif (10) selon la revendication 1, dans lequel le dispositif d'enfichage (3) comprend en outre un aimant permanent (8) qui est disposé coaxialement à l'électrode intérieure (4, 4a, 4b).

3. Dispositif (10) selon la revendication 1 ou 2, dans lequel
la base en forme de plaque (11) comprend en outre des éléments de montage (3d) qui sont disposés de manière optionnelle à un bord de la base en forme de plaque (11).

4. Dispositif (10) selon l'une quelconque des revendications 1 à 3, dans lequel
la base en forme de plaque (11) comprend un élément de retenue de photodiode (12) qui fait saillie à partir d'une première surface (13) de la base en forme de plaque (11), et
le dispositif d'enfichage (3) comprend en outre une photodiode (9) pour la surveillance la décharge barrière atmosphérique, qui est disposée à l'élément de retenue de photodiode (12) et qui est exposée vers l'extérieur.

5. Dispositif (10) selon l'une quelconque des revendications 1 à 3, dans lequel
la base en forme de plaque (11) comprend un élément de retenue de dispositif émettant des UV-C (12) qui fait saillie à partir d'une première surface (13) de la base en forme de plaque (11), et
le dispositif d'enfichage (3) comprend en outre un dispositif émettant des UV-C (9) qui est disposé à l'élément de retenue de dispositif émettant des UV-C (12) et qui est exposé vers l'extérieur.

6. Dispositif (10) selon l'une quelconque des revendications 1 à 5, dans lequel l'électrode intérieure (4, 4a) est formée d'un élastomère conducteur résistant à la température.

7. Dispositif (10) selon la revendication 6, dans lequel l'électrode intérieure (4, 4a) est pressée contre la surface d'enveloppe intérieure (2a) au moyen du premier élément de retenue (3a).

8. Dispositif (10) selon l'une quelconque des revendications 1 à 7, dans lequel
le premier élément de retenue (3a, 3b) comporte un trou traversant (18),
le dispositif (10) comprend une ligne de raccordement à tension alternative (6) qui est reliée électriquement à l'électrode intérieure (4, 4a, 4b), et
la ligne de raccordement à tension alternative (6) passe à travers le trou traversant (18) du premier élément de retenue (3a, 3b).

9. Dispositif (10) selon l'une quelconque des revendications 1 à 8, dans lequel
le dispositif (10) comprend une ligne de raccordement à masse (5) qui est reliée électriquement à l'électrode extérieure (1),
ledit au moins un moyen de retenue (3a, 3b, 3c) comprend un deuxième élément de retenue (3c) qui fait saillie à partir de la première surface (13) de la base en forme de plaque (11), et
le deuxième moyen de retenue (3c) est configuré pour guider mécaniquement la ligne de raccordement à masse (5).

10. Dispositif (10) selon l'une quelconque des revendications 1 à 9, dans lequel
l'électrode extérieure (1) est formée comme cylindre creux et comprend des fils transversaux (1b) ainsi que des fils longitudinaux (1a),
les fils transversaux (1b) sont formés d'un matériau conducteur, et
les fils longitudinaux (1a) sont formés d'un matériau conducteur avec un diamètre de fil qui est de 10 % à 15 % plus fin qu'un diamètre de fil des fils transversaux (1b).

11. Dispositif (10) selon la revendication 10, dans lequel
les fils transversaux (1b) et les fils longitudinaux (1a) forment le tissu à mailles rectangulaires (21), et
une distance entre des fils transversaux (1b) adjacents est comprise entre 4 et 6 fois le diamètre des fils longitudinaux (1a),
dans lequel, de manière optionnelle, une distance d'un fil transversal extrême (19), qui est disposé dans une partie de bord (20) du tissu à mailles rectangulaires (21) dans une direction longitudinale du tissu à mailles rectangulaires (21), à une extrémité libre (30) respective des fils longitudinaux (1a) dans la partie de bord (20) est comprise entre 0,8 et 1,5 fois la distance des fils transversaux (1b) adjacents du tissu à mailles rectangulaires (21).

12. Utilisation d'un dispositif (10) selon l'une quelconque des revendications précédentes pour montage d'enfichage du dispositif (10) au moyen du dispositif d'enfichage (3) dans une ouverture prédéfinie à un réacteur.

13. Procédé pour faire fonctionner un dispositif (10) pour décharge barrière atmosphérique selon l'une quelconque des revendications 1 à 11, pourvu qu'en combinaison avec la revendication 4, le procédé comprenant:
appliquer une tension alternative ayant une valeur de tension et une fréquence entre l'électrode intérieure (4, 4a, 4b) et l'électrode extérieure (1) pour générer une décharge barrière atmosphérique à l'électrode extérieure (1);
mesurer une intensité UV de la décharge barrière atmosphérique générée au moyen de la photodiode (9);
commander la valeur de tension et la fréquence à base de l'intensité UV mesurée, de sorte que le dispositif (10) de décharge barrière atmosphérique fonctionne dans une zone d'un état de résonance électrique.

14. Procédé selon la revendication 13, qui comprend en outre:
commander la valeur de tension et la fréquence de manière à ce qu'un plasma à dominante ozone et un plasma à dominante azote soient générés simultanément et séparément l'un de l'autre dans le dispositif (10) pour décharge barrière atmosphérique.

15. Procédé selon la revendication 13 ou 14, qui comprend en outre:
faire fonctionner le dispositif (10) pour décharge barrière atmosphérique à un réacteur (par exemple une armoire de stérilisation);
alimenter de l'air filtré de l'extérieur du réacteur à travers le dispositif (10) pour décharge barrière atmosphérique;
traiter par plasma l'air alimenté au moyen du dispositif (10) pour décharge barrière atmosphérique;
alimenter le réacteur en air traité par plasma.
